# EUROPÄISCHE PATENTANMELDUNG

(11) **EP 1 738 797 A2**
(43) Veröffentlichungstag der Anmeldung: **03.01.2007**
(21) Anmeldenummer: 06012547.3
(22) Anmeldetag: 19.06.2006
(51) Int. Cl.: A61N 5/06

(54) **Klimatisiertes Bräunungsgerät**

(30) Priorität: 29.06.2005 DE 102005030388
(71) Anmelder: JK-Holding GmbH, 53578 Windhagen (DE)
(72) Erfinder: Kratz, Walter, 3620 Lanaken-Neerharen (BE)
(74) Vertreter: Koepe, Gerd L.

(57) **Zusammenfassung**

Die Erfindung betrifft ein Bräunungsgerät, insbesondere zur Ganzkörper-Bräunung eines Benutzers, umfassend ein standfestes Unterteil mit elektrischen Aggregaten, Schalt-Einrichtungen und Verbindungs-Einrichtungen; und ein entweder an einer der Längsseiten des Unterteils über Scharniere oder Gelenke angebrachtes, über die Scharniere oder Gelenke von dem Unterteil weg und auf das Unterteil hin zur Bildung eines im wesentlichen tunnelförmigen Bräunungsraumes klappbares oder schwenkbares oder an einer separaten Aufhängung von dem Unterteil weg hebbares und auf das Unterteil hin zur Bildung eines im wesentlichen tunnelförmigen Bräunungsraumes senkbares Oberteil; wobei das Unterteil eine für UV-Strahlung durchlässige Liegefläche für den Benutzer und unterhalb der Liegefläche mindestens einen Satz in Längsrichtung des Bräunungsgeräts angeordneter Strahler zur Abgabe von bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und das Oberteil eine für zumindest bräunende UV-Strahlung durchlässige Schutzfläche und oberhalb der Schutzfläche mindestens einen Satz in Längsrichtung des Bräunungsgeräts angeordneter Strahler zur Abgabe von bräunender UV-Strahlung in Richtung auf das Unterteil und/oder zumindest einen Teil des Körpers des Benutzers gerichtet aufweist; und wobei das Unterteil entlang zumindest eines Teils der Längsseiten der Liegefläche Ausströmer-Düsen und/oder -Kanäle aufweist, die im wesentlichen in Richtung auf die Schutzfläche des Oberteils gerichtet sind und in den Bräunungsraum einen Luftstrom einspeisen, der den Körper des Benutzers nach Durchströmen des Bräunungsraums temperiert. Die Erfindung betrifft auch ein Verfahren zum Temperieren eines Bräunungsraums eines Bräunungsgeräts und zum Temperieren des Körpers eines das in dem Bräunungsgerät liegenden Benutzers.

## Beschreibung

Die vorliegende Erfindung betrifft ein Bräunungsgerät mit Klimatisierung. Insbesondere betrifft die vorliegende Erfindung ein Bräunungsgerät, dessen Klimatisierung weitgehend über Aggregate erfolgt, die im Unterteil des Bräunungsgeräts angeordnet werden können.

Geräte zur Körperbräunung, insbesondere Geräte zur Bräunung im wesentlichen des gesamten Körpers, sind im Stand der Technik umfangreich beschrieben. Sie bestehen in der Regel aus einem standfesten Unterteil, das es aufgrund seines Gesamtvolumens erlaubt, im wesentlichen die gesamte Technik des Geräts, insbesondere die elektrischen Aggregate, Schalt- und Verbindungs-Einrichtungen, unterzubringen, und einem regelmäßig an einer der Längsseiten des Unterteils über Scharniere oder Gelenke angebrachten, um diese Scharniere bzw. Gelenke von dem Unterteil weg und auf dieses hin zur Bildung eines Bräunungsraums klappbaren oder schwenkbaren Oberteil. Das Oberteil kann in Ausführungsformen des Bräunungsgeräts auch von dem Unterteil unabhängig an einer separaten Aufhängung hebbar und senkbar aufgehängt sein; die Aufhängung ist regelmäßig entweder an einem auf dem mit dem Unterteil gemeinsamen Boden stehenden oder an einer Wand angebrachten Arm oder an einer an der Decke angebrachten Vorrichtung verankert, wobei die Wand oder die Decke Teile des Raums sind, in dem das Bräunungsgerät aufgestellt ist.

Oberhalb des für die Unterbringung der Technik geeigneten Raums des Unterteils findet sich ein Satz oder befinden sich mehrere Sätze in Längsrichtung des Bräunungsgeräts angeordneter Strahler, beispielsweise Strahlung mit einem bräunenden Anteil UV-Strahlung emittierender Röhren oder Hochdruck-Strahler, deren Strahlung im wesentlichen in Richtung auf das Oberteil des Bräunungsgeräts auf einen Teil des Körpers eines Benutzers abgegeben wird, der auf einer transparenten, zumindest für die von den Strahlerröhren abgegebene UV-Strahlung durchlässigen Liegefläche liegt. Die Strahler können so beschaffen sein, daß sie Strahlung zum Bräunen aller Partien des Körpers des Benutzers abgeben, oder können so beschaffen sein, daß sie Strahlung zum Bräunen nur bestimmter Partien des Körpers des Benutzers abgeben, beispielsweise der Beine, des Rumpfes und/oder der Schulter.

In entsprechender Weise findet sich im Oberteil (im Betriebszustand) oberhalb einer dem Benutzer zugewandten, weitgehend parallel zu Liegefläche des Unterteils angeordneten und für UV-Strahlung durchlässigen Schutzfläche mindestens ein Satz in Längsrichtung des Bräunungsgeräts angeordneter Strahler, beispielsweise Strahlung mit einem bräunenden Anteil UV-Strahlung emittierender Röhren oder Hochdruck-Strahler, deren Strahlung im wesentlichen in Richtung auf das Unterteil des Bräunungsgeräts auf einen Teil des Körpers des Benutzers abgegeben wird. In einer bevorzugten Ausführungsform finden sich im Oberteil des Bräunungsgeräts mindestens zwei Sätze von bräunende UV-Strahlung angebenden Strahlern, von denen beispielsweise einer der Emission von UV-Strahlung auf den Körper des Benutzers und der zweite einer Emission von UV-Strahlung auf das Gesicht des Benutzers dient.

Neben UV-Strahlung und sichtbarer Strahlung emittieren die UV-Strahlerröhren und Hochdruck-Strahler bei Betrieb eine merkliche Menge infraroter Strahlung, die vom Benutzer natürlicherweise als Wärme empfunden wird. Die Wärmestrahlung, in der kalten Jahreszeit und zu Beginn des Bräunungsvorgangs noch (individuell unterschiedlich) als angenehm empfunden, erreicht jedoch im weiteren Fortgang des Bräunungsvorgangs im Innern des Bräunungsgeräts und insbesondere in den warmen Jahreszeiten unangenehme Werte. Aus diesem Grund werden Bräunungsgeräte bereits im Stand der Technik mit Kühl- und Lüftungs-Einrichtungen ausgestattet.

So beschreibt beispielsweise der Stand der Technik ein typisches Bräunungsgerät mit Lüftung bzw. Kühlung, deren Lüfter über das gesamte Gerät verteilt sind, um möglichst viel Frischluft anzusaugen und über isolierte Kanäle Ausström-Düsen zuzuführen, die sich ausschließlich im Oberteil des Bräunungsgeräts befinden. In einem (aufgrund der Technik voluminösen und daher nur im Unterteil des Bräunungsgerät unterbringbaren) Klima-Kühler erzeugte Kaltluft wird nach individuellem Bedarf der Frischluft zudosiert, was eine weitere Isolation von Kühlleitungen erfordert: Die Erfahrung zeigt, daß mit diesen Einrichtungen lediglich die Kühlung von Luft erreicht wird, die dann auf den Körper des Benutzers des Bräunungsgeräts aufgeblasen wird. Eine gleichmäßige Kühlung des Bräunungsraums, d. h. des Raums zwischen Unterteil und Oberteil des Bräunungsgeräts, in dem sich der Benutzer des Bräunungsgeräts während des Bräunungsraums aufhält, wird dadurch nicht erreicht. Dies wird nicht immer als angenehm, im Einzelfall sogar wegen des entstehenden Zuges als unangenehm empfunden und stört das Bräunungserlebnis nachhaltig. Darüber hinaus sind die Ausström-Düsen für die Gesichtskühlung immer links und rechts direkt neben dem Kopf angeordnet, müssen aufwendig ― je nach Person und Empfinden unterschiedlich ― eingestellt werden. Sie blasen dann die kühle Luft auf die Ohren, die Nase und den Mund des Benutzers. Das direkte Anblasen der Ohren erzeugt unangenehm hohe Luftströmungs-Geräusche, die ein entspanntes Bräunen unmöglich machen und zudem das störungsfreie Aufnehmen akustischer Signale (z. B. Musik) verhindern.

Zu Beginn des Bräunungsvorgangs legt sich der Benutzer auf die Liegefläche des Bräunungsgeräts, die regelmäßig aus einem mehr oder weniger glatten, für zumindest bräunende UV-Strahlung durchlässigen Kunststoff-Material besteht. Das Material wird vom Körper des Benutzers als kalt empfunden, zumindest dann, wenn das Gerät vor dem in Frage stehenden Bräunungsvorgang noch nicht in Betrieb war oder die bräunende Strahlung abgebenden Strahler oder Brenner längere Zeit ausgeschaltet waren. Eine Vorwärmung der Liegefläche und gegebenenfalls auch des Bräunungsraums des Bräunungsgeräts könnte daher den Komfort des Benutzers verbessern. Im Stand der Technik werden zur Vorwärmung der Liegefläche bereits Vorschläge gemacht. Verwiesen wird beispielsweise auf die Druckschrift DE-A 100 53 900.9.

Generell ist also eine Temperierung, bevorzugt eine Kühlung, des Inneren eines Bräunungsgeräts und insbesondere des Bräunungsraums eines Bräunungsgeräts angestrebt.

Überraschend wurde nun gefunden, daß eine gleichmäßige Temperierung und bevorzugterweise Kühlung des gesamten Bräunungsraums und gegebenenfalls der Liegefläche erreicht werden kann, ohne daß dazu notwendige Technologie (z. B. (gegebenenfalls isolierte) Leitungen, Düsen, Düsensteuerung usw.) das Oberteil des Bräunungsgeräts gewichtsmäßig belastet, wenn man die kühlende Luft über längs der Liegefläche am Unterteil des Bräunungsgeräts angeordnete Ausströmer-Düsen heranführt.

Die Erfindung betrifft daher ein Bräunungsgerät, insbesondere zur Ganzkörper-Bräunung eines Benutzers, das umfaßt:
- ein standfestes Unterteil, enthaltend elektrische Aggregate, Schalt-Einrichtungen und Verbindungs-Einrichtungen; und
- ein entweder an einer der Längsseiten des Unterteils über Scharniere oder Gelenke angebrachtes, über die Scharniere oder Gelenke von dem Unterteil weg und auf das Unterteil hin zur Bildung eines im wesentlichen tunnelförmigen Bräunungsraumes klappbares oder schwenkbares oder an einer separaten Aufhängung von dem Unterteil weg hebbares und auf das Unterteil hin zur Bildung eines im wesentlichen tunnelförmigen Bräunungsraumes senkbares Oberteil; wobei
- das Unterteil eine für zumindest bräunende UV-Strahlung durchlässige Liegefläche für den Benutzer und unterhalb der Liegefläche mindestens einen Satz in Längsrichtung des Bräunungsgeräts angeordneter Strahler zur Abgabe von Strahlung mit zumindest einem Anteil an bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und
- das Oberteil eine für zumindest bräunende UV-Strahlung durchlässige Schutzfläche für den Benutzer und oberhalb der Schutzfläche mindestens einen Satz in Längsrichtung des Bräunungsgeräts angeordneter Strahler zur Abgabe von Strahlung mit zumindest einem Anteil an bräunender UV-Strahlung in Richtung auf das Unterteil und/oder zumindest einen Teil des Körpers des Benutzers gerichtet aufweist; und wobei
- das Unterteil entlang zumindest eines Teils der Längsseiten der Liegefläche Ausströmer-Düsen oder Ausströmer-Kanäle aufweist, die im wesentlichen in Richtung auf die Schutzfläche des Oberteils gerichtet sind und in den Bräunungsraum einen Luftstrom einspeisen, der den Körper des Benutzers nach Durchströmen des Bräunungsraums temperiert.

Bevorzugte Ausführungsformen des erfindungsgemäßen Bräunungsgeräts sind in den abhängigen Ansprüchen 2 bis 11 beansprucht.

Die Erfindung betrifft auch ein Bräunungsgerät, insbesondere zur Ganzkörper-Bräunung eines Benutzers, das umfaßt:
- ein standfestes Unterteil, enthaltend elektrische Aggregate, Schalt-Einrichtungen und Verbindungs-Einrichtungen; und
- ein entweder an einer der Längsseiten des Unterteils über Scharniere oder Gelenke angebrachtes, über die Scharniere oder Gelenke von dem Unterteil weg und auf das Unterteil hin zur Bildung eines im wesentlichen tunnelförmigen Bräunungsraumes klappbares oder schwenkbares oder an einer separaten Aufhängung von dem Unterteil weg hebbares und auf das Unterteil hin zur Bildung eines im wesentlichen tunnelförmigen Bräunungsraumes senkbares Oberteil; wobei
- das Unterteil eine für zumindest bräunende UV-Strahlung durchlässige Liegefläche für den Benutzer und unterhalb der Liegefläche mindestens einen Satz in Längsrichtung des Bräunungsgeräts angeordneter Strahler zur Abgabe von Strahlung mit zumindest einem Anteil an bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und
- das Oberteil eine für zumindest bräunende UV-Strahlung durchlässige Schutzfläche für den Benutzer und oberhalb der Schutzfläche mindestens einen Satz in Längsrichtung des Bräunungsgeräts angeordneter Strahler zur Abgabe von Strahlung mit zumindest einem Anteil an bräunender UV-Strahlung in Richtung auf das Unterteil und/oder zumindest einen Teil des Körpers des Benutzers gerichtet aufweist; und wobei
- das Unterteil zwischen Liegefläche und dem mindestens einen Satz Strahler zur Abgabe von Strahlung mit zumindest einem Anteil an bräunender UV-Strahlung eine für zumindest bräunende UV-Strahlung durchlässige Zwischenfläche aufweist, die im wesentlichen in einer Ebene parallel zur Ebene der Liegefläche verläuft und die Führung von Luft im Raum zwischen der Zwischenfläche und der Liegefläche zur Temperierung der letzteren erlaubt.

Bevorzugte Ausführungsform des Bräunungsgeräts entsprechend dieser Ausführungsform sind in den abhängigen Ansprüchen 13 bis 22 beansprucht.

Die Erfindung betrifft in einer weiteren Ausführungsform auch ein Bräunungsgerät, insbesondere zur Ganzkörper-Bräunung eines Benutzers, das umfaßt:
- ein standfestes Unterteil, enthaltend elektrische Aggregate, Schalt-Einrichtungen und Verbindungs-Einrichtungen; und
- ein entweder an einer der Längsseiten des Unterteils über Scharniere oder Gelenke angebrachtes, über die Scharniere oder Gelenke von dem Unterteil weg und auf das Unterteil hin zur Bildung eines im wesentlichen tunnelförmigen Bräunungsraumes klappbares oder schwenkbares oder an einer separaten Aufhängung von dem Unterteil weg hebbares und auf das Unterteil hin zur Bildung eines im wesentlichen tunnelförmigen Bräunungsraumes senkbares Oberteil; wobei
- das Unterteil eine für zumindest bräunende UV-Strahlung durchlässige Liegefläche für den Benutzer und unterhalb der Liegefläche mindestens einen Satz in Längsrichtung des Bräunungsgeräts angeordneter Strahler zur Abgabe von Strahlung mit zumindest einem Anteil an bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und
- das Oberteil eine für zumindest bräunende UV-Strahlung durchlässige Schutzfläche für den Benutzer und oberhalb der Schutzfläche mindestens einen Satz in Längsrichtung des Bräunungsgeräts angeordneter Strahler zur Abgabe von Strahlung mit zumindest einem Anteil an bräunender UV-Strahlung in Richtung auf das Unterteil und/oder zumindest einen Teil des Körpers des Benutzers gerichtet aufweist; und wobei
- das Unterteil entlang zumindest eines Teils der Längsseiten der Liegefläche Ausströmer-Düsen oder Ausströmer-Kanäle aufweist, die im wesentlichen in Richtung auf die Schutzfläche des Oberteils gerichtet sind und in den Bräunungsraum einen Luftstrom einspeisen, der den Bräunungsraum auch bei nicht arbeitenden Strahlern temperiert.

Bevorzugte Ausführungsformen des Bräunungsgeräts gemäß dieser Ausführungsform der Erfindung sind in den abhängigen Ansprüchen 24 bis 35 beansprucht.

Gemäß den in den abhängigen Ansprüchen 36 und 37 beanspruchten Ausführungsformen der Erfindung besteht die Temperierung in einer Erwärmung oder vorzugsweise einer Kühlung des Luftstroms, noch weiter bevorzugt gegenüber der das Bräunungsgerät umgebenden Luft.

Die Erfindung betrifft auch ein Verfahren zum Temperieren eines Bräunungsraums eines Bräunungsgeräts bzw. ein Verfahren zum Temperieren des Körpers eines Benutzers in einem Bräunungsgerät mit
- einem standfesten Unterteil, enthaltend elektrische Aggregate, Schalt-Einrichtungen und Verbindungs-Einrichtungen; und
- einem entweder an einer der Längsseiten des Unterteils über Scharniere oder Gelenke angebrachten, über die Scharniere oder Gelenke von dem Unterteil weg und auf das Unterteil hin zur Bildung eines im wesentlichen tunnelförmigen Bräunungsraumes klappbaren oder schwenkbaren oder an einer separaten Aufhängung von dem Unterteil weg hebbaren und auf das Unterteil hin zur Bildung eines im wesentlichen tunnelförmigen Bräunungsraumes senkbaren Oberteil; wobei
- das Unterteil eine für zumindest bräunende UV-Strahlung durchlässige Liegefläche für den Benutzer und unterhalb der Liegefläche mindestens einen Satz in Längsrichtung des Bräunungsgeräts angeordneter Strahler zur Abgabe von Strahlung mit zumindest einem Anteil an bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und
- das Oberteil eine für zumindest bräunende UV-Strahlung durchlässige Schutzfläche für den Benutzer und oberhalb der Schutzfläche mindestens einen Satz in Längsrichtung des Bräunungsgeräts angeordneter Strahler zur Abgabe von Strahlung mit zumindest einem Anteil an bräunender UV-Strahlung in Richtung auf das Unterteil und/oder zumindest einen Teil des Körpers des Benutzers gerichtet aufweist;
- bei dem man mittels entlang zumindest der Längsseiten der Liegefläche des Unterteils des Bräunungsgeräts angeordneter Ausströmer-Düsen oder Ausströmer-Kanäle im wesentlichen in Richtung auf die Schutzfläche des Oberteils des Bräunungsgeräts in den Bräunungsraum einen Luftstrom einspeist, der den Bräunungsraum bei Durchströmen temperiert; bzw.
- bei dem man mittels entlang zumindest der Längsseiten der Liegefläche des Unterteils des Bräunungsgeräts angeordneter Ausströmer-Düsen oder Ausströmer-Kanäle im wesentlichen in Richtung auf die Schutzfläche des Oberteils des Bräunungsgeräts in den Bräunungsraum einen Luftstrom einspeist, der den Körper des Benutzers nach Durchströmen des Bräunungsraums temperiert.

Bevorzugte Ausführungsformen der Verfahren sind in den abhängigen Ansprüchen 40 und 41 beansprucht.

Die Erfindung wird nachfolgend anhand der beigefügten Figuren näher erläutert, wobei die Figuren und deren detaillierte Beschreibung nur der beispielhaften Erläuterung der Erfindung anhand ihrer bevorzugten Ausführungsformen dienen und die Erfindung keinesfalls beschränken. Insbesondere ist es möglich, verschiedene Merkmale der Erfindung miteinander zu kombinieren, ohne daß die Kombinationsmöglichkeiten irgendwelchen Beschränkungen unterlegen. Es zeigen:

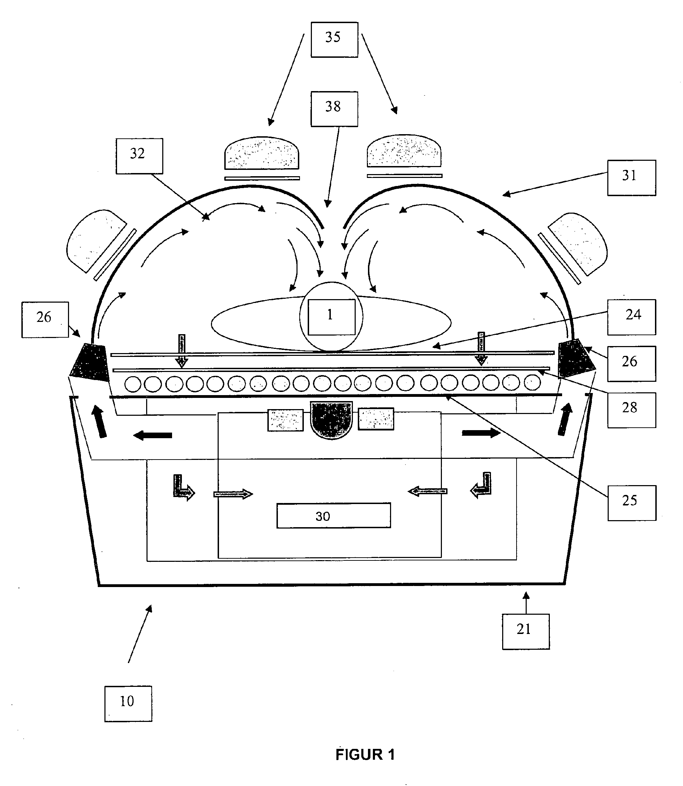

Figur 1 eine Schnittansicht des Bräuners gemäß der Erfindung von der Kopfseite gesehen;

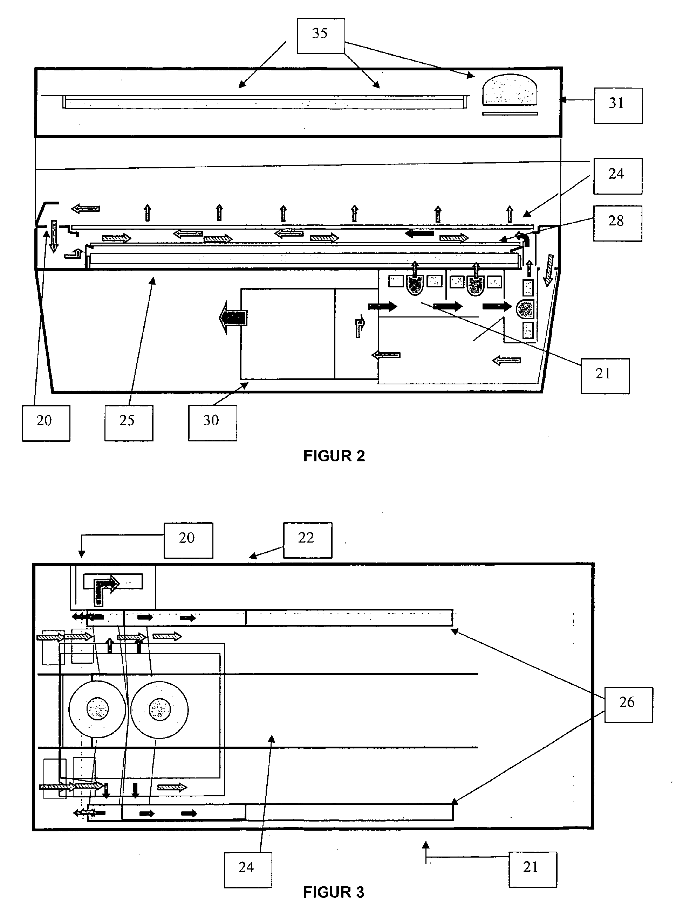

Figur 2 eine Schnittansicht des Bräuners gemäß der Erfindung von einer Längsseite gesehen;
Figur 3 eine Aufsicht auf eine Ausführungsform des Bräuners der Erfindung; und

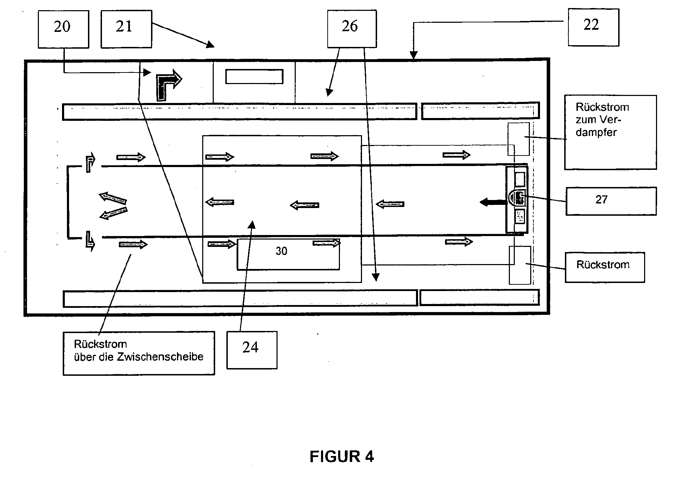

Figur 4 eine Aufsicht auf eine weitere Ausführungsform des Bräuners der Erfindung.

Das erfindungsgemäße Bräunungsgerät ist in den Figuren mit dem Bezugszeichen 10 bezeichnet und dient vorzugsweise der Bräunung des gesamten Körpers eines Benutzers 1 mittels künstlich erzeugter Strahlung, die zumindest einen Teil an aktinischer Strahlung im UV-Bereich (λ ≤ 400 nm) enthält, der auf den Körper eines Benutzers 1 bräunend wirkt, also die Melanin-Bildung in der Haut des Benutzers 1 anregt. Im Rahmen der Erfindung liegen jedoch auch Bräunungsgeräte 10, die der Bräunung des gesamten Körpers eines Benutzers 1 dienen können, jedoch zumindest zeitweise nur zur Bräunung einzelner Teile des Körpers eines Benutzers 1 verwendet werden, beispielsweise zur Bräunung des Gesichts und/oder des Oberkörpers eines Benutzers 1.

Solche Bräunungsgeräte sind als solche aus dem Stand der Technik bekannt und bestehen regelmäßig aus mehreren Teilen, die sich in der Mehrzahl der Fälle ― jedoch nicht ausschließlich ― entweder einem standfesten Unterteil 21 und einem bewegbaren Oberteil 31 zuordnen lassen.

Das Unterteil 21 dient in der Regel der Unterbringung der für den Betrieb des Bräunungsgeräts 10 erforderlichen elektrischen Aggregate, Schalteinrichtungen und elektrischen, mechanischen und strömungstechnischen Verbindungseinrichtungen. Diese lassen sich mehr oder weniger erschütterungsfrei in dem relativ großvolumigen Unterteil 21 unterbringen. In vielen Fällen ist es bevorzugt, im Unterteil 21 auch eine Einrichtung oder Einrichtungen zum Temperieren einzelner Bereiche und/oder Teile des Bräunungsgeräts und/oder zugehörige Gebläse-Einrichtungen unterzubringen. Im Stand der Technik führte dies immer wieder zu Problemen, wenn vom Unterteil 21 Versorgungsleitungen strömungstechnischer Art (beispielsweise zum Heranführen von Kühlungsluft) zum Oberteil 31 geführt werden mußten und diese Leitungen gegen Wärme- (oder besser: Kälte-) Verluste zu isolieren waren. Auch die Mehrzahl anderer zum Betrieb eines Bräunungsgeräts 10 erforderlicher Aggregate ist ― allein schon aus Gewichtsgründen ― im Unterteil des Bräunungsgeräts untergebracht.

Dem Unterteil 21 im wesentlichen in den Ausmaßen entsprechend ist bei den Bräunungsgeräten der eingangs genannten Art ein Oberteil 31 vorgesehen. Das Oberteil 31 ist ― regelmäßig und so auch erfindungsgemäß - entweder an einer der Längsseiten 22 des Unterteils 21 mittels Scharnieren oder Gelenken 23 angebracht und ist um diese Scharniere oder Gelenke 23 klappbar oder schwenkbar, und zwar von dem Unterteil 21 weg (unter Öffnen des Bräunungsgeräts 10), wodurch das "Einsteigen" des Benutzers 1 in das Bräunungsgerät 10 erleichtert wird, oder auf das Unterteil 21 hin. Durch einen Klapp- oder Schwenkvorgang des Oberteils 31 zu dem Unterteil 21 des Bräunungsgeräts 10 hin wird das Bräunungsgerät 10 vor Beginn des Bräunungsvorgangs geschlossen und so ein im wesentlichen tunnelförmiger Bräunungsraum 32 gebildet, in dem der Benutzer 1 liegt.

In einer alternativen Ausführungsform der Erfindung sind Unterteil 21 und Oberteil 31 des Bräunungsgeräts 10 nicht über Scharniere oder Gelenke 23 klappbar oder schwenkbar verbunden. Vielmehr ist das Oberteil 31 an einer separaten Aufhängung 33 befestigt, die entweder mit einem Arm an der Wand des Raums angebracht ist, in dem das Bräunungsgerät 10 aufgestellt ist, oder an einem auf dem Boden des Raums stehenden Ständer angebracht ist, in dem das Bräunungsgerät aufgestellt ist, oder an der Decke des Raums angebracht ist, in dem das Bräunungsgerät aufgestellt ist. Mittels der Aufhängung und einer geeigneten (gegebenenfalls elektronisch betriebenen) Mechanik kann das so befestigte Oberteil 31 von dem Unterteil 21 des Bräunungsgeräts weg (zum Erleichtern des "Einsteigens" des Benutzers 1) oder auf das Unterteil 21 hin bewegt werden. Die Bewegung des Oberteils 31 auf das Unterteil 21 des Bräunungsgeräts 10 hin führt ebenfalls zum Schließen des Bräunungsgeräts 10 vor Beginn des Bräunungsvorgangs und zur Bildung eines im wesentlichen tunnelförmigen Bräunungsraums 32, in dem der Benutzer 1 während des Bräunungsvorgangs liegt.

Das Unterteil 21 des Bräunungsgeräts 10 weist für den Benutzer 1 eine für zumindest bräunende UV-Strahlung durchlässige Liegefläche 24 auf. Regelmäßig ist die Liegefläche 24 für die gesamte Strahlung durchlässig, die in dem Bräunungsgerät erzeugt wird; sie kann jedoch auch eine bezüglich bestimmter Strahlung selektive Durchlässigkeit aufweisen; insbesondere kann dem Material der Liegefläche 24 eine Filterfunktion für den Körper des Benutzers 1 schädigende UV-Strahlung oder sichtbare Strahlung zugeordnet werden. Zur Erzielung eines Bräunungseffekts durch physiologische Melaninbildung ist es jedoch erforderlich, daß das Material der Liegefläche 24 zumindest für den bräunenden Anteil der UV-Strahlung hinreichend durchlässig ist; eine 100 %ige Durchlässigkeit ist nicht erforderlich, eine möglichst hohe Durchlässigkeit für den bräunenden Anteil der UV-Strahlung ist jedoch erwünscht und angestrebt.

In dem erfindungsgemäßen Bräunungsgerät 10 ist unterhalb der Liegefläche 24 mindestens ein Satz Strahler 25 zur Abgabe von Strahlung mit zumindest einem Anteil an bräunender UV-Strahlung angeordnet. Praktischerweise sind die Strahler 25 in Längsrichtung des Bräunungsgeräts 10 angeordnet, also in Richtung vom Kopfende auf das Fußende des Geräts hin. Die Strahler 25 können aus einem Satz Strahler 25 oder aus mehreren Sätzen Strahler 25 bestehen. Im letztgenannten Fall sind die Strahler 25 regelmäßig vorzugsweise so angeordnet, daß sie einzelne Partien des Körpers des Benutzers gesondert bestrahlen, beispielsweise die Beine, die Becken-Region, den Rücken, die Schulter oder auch mehrere der vorgenannten Körperpartien. Die Strahler geben die zumindest einen Anteil der bräunenden Strahlung umfassende Strahlung in Richtung auf das Oberteil 31 des Bräunungsgeräts 10 und/oder zumindest auf einen Teil des Körpers des auf der Liegefläche 24 liegenden Benutzers 1 ab. Es versteht sich für den Fachmann, daß die Strahler 25 mit den für ihren Betrieb erforderlichen elektrischen Aggregaten, (Vor-) Schalteinrichtungen und/oder Verbindungs-Einrichtungen direkt oder indirekt verbunden sind. Solche Verbindungen und Schaltungen sind als solche aus dem Stand der Technik bekannt und bedürfen an dieser Stelle keiner weiteren Erläuterung.

In dem Unterteil 21 des Bräunungsgeräts 10 vergleichbarer Weise weist des Oberteil auf seiner unteren (d. h. beim Betrieb dem Benutzer zugewandten) Seite eine für zumindest bräunende UV-Strahlung durchlässige Schutzfläche 34 auf. Die Schutzfläche 34 ist aus Gründen der Gewichtsersparnis aus einem möglichst leichten, die UV-Durchlässigkeit garantierenden Material, dem ― wie bei der Liegefläche 24 des Unterteils 21 des Bräunungsgeräts 10 ― gegebenenfalls auch die Funktion eines Filters für Licht bestimmter Wellenlängen zugeordnet werden kann. Zur Erzielung eines Bräunungseffekts durch physiologische Melaninbildung ist es jedoch erforderlich, daß das Material der Schutzfläche 34 zumindest für den bräunenden Anteil der UV-Strahlung hinreichend durchlässig ist; eine 100 %ige Durchlässigkeit ist nicht erforderlich, eine möglichst hohe Durchlässigkeit für den bräunenden Anteil der UV-Strahlung ist jedoch erwünscht und angestrebt.

In dem erfindungsgemäßen Bräunungsgerät 10 ist oberhalb der Schutzfläche 34 mindestens ein Satz Strahler 35 zur Abgabe von Strahlung mit zumindest einem Anteil an bräunender UV-Strahlung angeordnet. Praktischerweise sind die Strahler 35 in Längsrichtung des Bräunungsgeräts 10 angeordnet, also in Richtung vom Kopfende auf das Fußende des Geräts hin. Die Strahler 35 können aus einem Satz Strahler 35 oder aus mehreren Sätzen Strahler 35 bestehen. Im letztgenannten Fall sind die Strahler 35 regelmäßig vorzugsweise so angeordnet, daß sie einzelne Partien des Körpers des Benutzers gesondert bestrahlen, beispielsweise die Beine, die Becken-Region, den Bauch, die Brust, das Gesicht oder auch mehrere der vorgenannten Körperpartien. Die Strahler geben die zumindest einen Anteil der bräunenden Strahlung umfassende Strahlung in Richtung auf das Unterteil 21 des Bräunungsgeräts 10 und/oder zumindest auf einen Teil des Körpers des auf der Liegefläche 24 liegenden Benutzers 1 ab. Es versteht sich für den Fachmann, daß die Strahler 35 mit den für ihren Betrieb erforderlichen elektrischen Aggregaten, (Vor-) Schalteinrichtungen und/oder Verbindungs-Einrichtungen direkt oder indirekt verbunden sind. Solche Verbindungen und Schaltungen sind als solche aus dem Stand der Technik bekannt und bedürfen an dieser Stelle keiner weiteren Erläuterung.

In einer Ausführungsform der Erfindung weist das Unterteil 21 entlang zumindest eines Teils der Längsseiten 22a, 22b der Liegefläche 24 Düsen oder Kanäle 26 auf, durch die Luft ausströmen kann, wobei die Ausströmer-Düsen 26 und/oder Ausströmer-Kanäle 26 im wesentlichen in Richtung auf die Schutzfläche 34 des Oberteils 31 des Bräunungsgeräts gerichtet sind und in den Bräunungsraum 32 des Bräunungsgeräts 10 einen Luftstrom einspeisen, der nach Durchströmen des Bräunungsraums 34 den Körper des Benutzers 1 temperiert.

Unter dem Begriff "Temperieren" bzw. "temperiert" wird im Rahmen der vorliegenden Erfindung und in den Patentansprüchen verstanden, daß durch technische Maßnahmen erreicht wird, daß sich die Temperatur des Bräunungsgeräts 10, eines Teils oder mehrerer Teile davon, wie beispielsweise (nicht beschränkend) der Liegefläche 24, der Kopfstütze (nicht gezeigt), der Fußstütze (nicht gezeigt), und/oder eines Bereichs davon, wie beispielsweise (nicht beschränkend) des Bräunungsraums 32, von einem bestimmten Wert T₁, der sich infolge bestimmter Verhältnisse wie beispielsweise der Umgebungstemperatur des Raumes, in dem das Bräunungsgerät 10 steht, einstellt, zu einem bestimmten anderen Wert T₂ ändert, den der Benutzer des Bräunungsgeräts 10 wünscht oder der allgemein von den Benutzern als angenehm empfunden wird. Dies kann ― beispielsweise ― der Fall sein, wenn der Betrieb des Bräunungsgeräts 10 durch Wärme-Emission der Strahler 25, 35 größere Mengen Wärme erzeugt und diese Wärme für den Benutzer unangenehm ist, so daß er eine Kühlung wünscht. In einem solchen Fall wird unter "Temperieren" eine Absenkung der Temperatur bzw. ein Kühlen verstanden. Ein anderer Fall des Kühlens ist die Situation, daß ein Bräunungsgerät 10 im Sommer (oder in warmen Gegenden) nicht in einem klimatisierten Raum steht und bereits bei Beginnen des Bräunungsvorgangs eine Temperatur herrscht, die über dem für den Benutzer angenehmen Wert liegt. Auch in einem solchen Fall wird unter "Temperieren" eine Absenkung der Temperatur bzw. ein Kühlen verstanden. Ein weiterer Fall des Kühlens besteht darin, daß das Bräunungsgerät 10 über einen gewissen Zeitraum in Benutzung war und die Liegefläche 24 durch die Wärmestrahlung der Strahler 25 auf eine Temperatur erwärmt ist, die über dem für den nächsten Benutzer angenehmen Wert liegt. Auch in einem solchen Fall wird unter Temperieren eine Absenkung der Temperatur bzw. ein Kühlen verstanden. Im Gegensatz dazu wird unter "Temperieren" ein Anheben der Temperatur bzw. ein Erwärmen verstanden, wenn das Bräunungsgerät 10 über längere Zeit nicht in Betrieb war und/oder in einem klimatisierten (oder kühlen bzw. gekühlten) Raum steht, so daß es für den Benutzer unangenehm ist, sich auf die kalte Liegefläche 24 oder in den kalten Bräunungsraum 32 zu legen, was beispielsweise im Winter passieren könnte. Die erfindungsgemäß bevorzugte Temperierung ist in diesem Fall die Kühlung. Weiter bevorzugt ist bei dem erfindungsgemäßen Bräunungsgerät, daß die Temperierung regelbar ist, vorzugsweise daß die Temperierung über eine Wärmund/oder Kühleinrichtung 27a und eine Einrichtung 27b zum Zudosieren von Umgebungsluft zu einem umlaufenden Luftstrom regelbar ist.

Mit im Unterteil 21 angeordneten Gebläse-Einrichtungen (nicht gezeigt) und Temperier-Einrichtungen (nicht gezeigt) über geeignete Leitungen verbundene Ausströmer-Düsen 26 oder Ausströmer-Kanäle 26 sind entlang zumindest eines Teils der Längsseiten 22a, 22b der Liegefläche 24, also vorzugsweise beidseitig der Liegefläche 24, angeordnet. Sie können aus einzelnen Düsen 26, also im wesentlichen runden oder ovalen Öffnungen, oder aus länglichen Kanälen 26 bestehen. Diese sind entweder konzentriert an bestimmten Stellen entlang der Längsseiten 22a, 22b der Liegefläche 24 angeordnet, um beispielsweise den Bräunungsraum 32 in Höhe bestimmter Stellen des Körpers des Benutzers 1, beispielsweise in Höhe des Gesichtes, stärker zu temperieren, oder sind gleichmäßig entlang einer der beiden (oder auch entlang beiden) Längsseiten 22a, 22b der Liegefläche 24 angeordnet, um eine gleichmäßige, keinen Zug erzeugende Temperierung des Bräunungsraums 32 zu erreichen. Es ist erfindungsgemäß besonders bevorzugt, daß die Ausströmer-Düsen 26 oder Ausströmer-Kanäle 26 gleichmäßig entlang der beiden Längsseiten 22a, 22b der Liegefläche angeordnet sind. Es ist erfindungsgemäß möglich, daß sich entlang der Längsseiten 22a, 22b der Liegefläche sowohl Ausströmer-Düsen als auch Ausströmer-Kanäle oder auch nur Ausströmer-Düsen 26 oder auch nur Ausströmer-Kanäle 26 finden, wobei die Reihenfolge und Anordnung vom Fachmann im wesentlichen frei und entsprechend den apparativen Erfordernissen frei gewählt werden kann. In einer weiteren bevorzugten Ausführungsform ist es auch möglich, daß entlang beider Längsseiten 22a, 22b der Liegefläche 24 über im wesentlichen die gesamte Länge auf jeder Seite der Liegefläche 24 je ein Ausströmer-Kanal 26 angeordnet ist.

Die Ausströmer-Düsen oder ―Kanäle 26 speisen in den Bräunungsraum 32 des Bräunungsgeräts 10 einen temperierten Luftstrom ein, beispielsweise einen kühlen Luftstrom. Dieser ist im wesentlichen in Richtung auf die Schutzfläche 34 der Oberteils 31 des Bräunungsgeräts 10 gerichtet. Hierunter wird verstanden, daß der Luftstrom nicht ―wie in herkömmlichen Bräunungsgeräten ― direkt auf den Körper des Benutzers gerichtet ist und eine direkte, oft als unangenehm empfundene und mitunter von störenden Luft-Strömungsgeräuschen begleitete Temperierung bzw. Kühlung des Körpers des Benutzers 1 bewirkt. Vielmehr strömt der Luftstrom in den Bräunungsraum 32 in Richtung auf dessen obere Begrenzung durch die Schutzfläche 34 des Oberteils 31 und erreicht des Körper des Benutzers 1 erst nach Durchströmen des Bräunungsraums 32. So wird überraschenderweise nicht nur das störende Luft-Strömungsgeräusch (beispielsweise an den Ohren des Benutzers) vermieden, sondern auch eine als sanft und angenehm empfundene Kühlung bewirkt, die vom Benutzer nicht als Zug erfahren wird.

In einer weiter bevorzugten Ausführungsform der Erfindung ist die für zumindest bräunende UV-Strahlung durchlässige Schutzfläche 34 des Oberteils 31 zur Bildung des in Längsrichtung des Bräunungsgeräts 10 im wesentlichen tunnelförmigen Bräunungsraums 32 aus Sicht des Benutzers konkav gewölbt. Die konkav gewölbte Schutzfläche ermöglicht in vorteilhafter Weise, daß der aus den Ausströmer-Düsen oder ―Kanälen 26 austretende Luftstrom entlang der konkaven Wölbung der Schutzfläche 34 strömen kann und dadurch gleichmäßig und gezielt wieder in den Bräunungsraum 32 zurückgeführt wird.

Erfindungsgemäß ist es weiter bevorzugt, daß die für zumindest bräunende UV-Strahlung durchlässige Schutzfläche 34 des Oberteils 31 des Bräunungsgeräts 10 im Scheitel 36 der konkaven Wölbung in Längsrichtung des Oberteils 31 einen im Querschnitt keilförmigen Luft-Umlenker 38 aufweist. Der Luft-Umlenker 38 ist weiter bevorzugt aus einem Material hergestellt, das ― wie die Schutzfläche 34 für den Benutzer 1 ― für zumindest bräunende UV-Strahlung durchlässig ist, um zu verhindern, daß diese Einrichtung 38 einen Teil der von den Strahlern 35 emittierten bräunenden UV-Strahlung absorbiert oder abschirmt. Der Luft-Umlenker 38 ist von besonderem Vorteil deswegen, weil sich die durch die Ausströmer-Düsen und/oder ―Kanäle 26 in den Bräunungsraum 32 eingespeiste Luft mit seiner Hilfe von der konkaven Wölbung der Schutzfläche 34 auf den Benutzer lenken läßt. In einer besonders bevorzugten Ausführungsform wird sogar die auf beiden Längsseiten 22a, 22b seitlich der Liegefläche durch die Ausströmer-Düsen und/oder ―Kanäle 26 einströmende Luft mittels des Luft-Umlenkers 38 in der Mitte des Bräunungsraums 32 verwirbelt, was zu einem als besonders angenehm empfundenen temperierenden Luftstrom am Körper des Benutzers 1 führt.

In einer noch weiter bevorzugten, mit besonderem Vorteil verwirklichten Ausführungsform der Erfindung ist der Luft-Umlenker 38 Teil der für zumindest bräunende UV-Strahlung durchlässigen Schutzfläche 34. In diesem Fall ist natürlich der Luft-Umlenker 38 aus demselben Material wie die Schutzfläche 34 und ist damit auch hinsichtlich seiner Durchlässigkeit für zumindest bräunende UV-Strahlung mit der Durchlässigkeit der Schutzfläche 34 identisch. Diese Ausführungsform ist auch aus Herstellungs-Sicht besonders vorteilhaft, da die Schutzfläche 34 zusammen mit dem Luft-Umlenker 38 integral gestaltet ist und entsprechend in einem Schritt hergestellt werden kann.

In einer dazu alternativen Ausführungsform kann der Luft-Umlenker 38 auf die für zumindest bräunende UV-Strahlung durchlässige Schutzfläche 34 aufgebracht werden, was natürlich auch nachträglich geschehen kann. Damit wird ein Nachrüsten der Schutzfläche 34 des Oberteils 32 des Bräunungsgeräts 10 mit dem Luft-Umlenker 38 möglich, so daß auch bereits laufenden Geräten die Vorteile der Lenkung des Luftstroms an der Schutzfläche 34 verliehen werden können. Die Aufbringung des Luft-Umlenkers 38 auf die Schutzfläche 34 kann auf jede beliebige, dem Fachmann bekannte Art und Weise erfolgen. Ganz besonders bevorzugt ist jedoch ein Aufkleben des Luft-Umlenkers 38 auf die Schutzfläche 34 des Oberteils 31 des Bräunungsgeräts 10.

Der Luft-Umlenker 38 auf der (oder integriert in die) für zumindest bräunende UV-Strahlung durchlässige Schutzfläche 34 kann eine völlig glatte Oberfläche aufweisen. Erfindungsgemäß ist es jedoch noch weiter bevorzugt, daß der Luft-Umlenker 38 eine der Verteilung und/oder Verwirbelung der strömenden Luft dienende Oberflächenstruktur 39 aufweist. Eine solche Oberflächenstruktur 39 kann zu einer noch besseren Verteilung des Luftstroms oder gar zu dessen Verwirbelung im Bräunungsraum 32 des Oberteils 31 des Bräunungsgeräts 10 führen, und die Temperierung des Bräunungsraums und des Körpers des Benutzers 1 erfolgen auf diesem Weg noch nachhaltiger und angenehmer. Die Oberflächenstruktur kann von beliebiger Art sein, wie sie der Fachmann für die angegebenen Zwecke kennt, ohne daß die Erfindung diesbezüglich irgendwelchen Beschränkungen unterliegt. In einer mit besonderem Vorteil verwirklichten Ausführungsform weist der Luft-Umlenker 38 Vertiefungen, Spiralen und/oder Wellen auf, die der Verteilung und/oder Verwirbelung der strömenden Luft dienen.

In einer alternativen oder auch zusammen mit der vorstehend beschriebenen Ausführungsform gemeinsam verwirklichten vorteilhaften Ausführungsform der Erfindung weist der Luft-Umlenker 38 Einrichtungen 40 auf, mit deren Hilfe die strömende Luft separat auf den Körper oder separat auf das Gesicht des Benutzers 1 geleitet werden kann. Solche Einrichtungen 40 sind dem Fachmann ebenfalls bekannt und können entweder statisch oder individuell einstellbar sein, damit in vorteilhafter Weise der Luftstrom auf die Stellen des Körpers oder des Gesichts gelenkt werden kann, die dem Benutzer 1 am angenehmsten sind.

In einer weiteren vorteilhaften Ausführungsform der Erfindung, die zusammen mit den vorstehenden Merkmalen des Bräunungsgeräts verwirklicht werden kann, weist das Unterteil 21 des Bräunungsgeräts 10 zwischen Liegefläche 24 und dem mindestens einen Satz Strahler 25 zur Abgabe von Strahlung mit zumindest einem Anteil an bräunender UV-Strahlung eine für zumindest bräunende UV-Strahlung durchlässige Zwischenfläche 28 auf, die im wesentlichen in einer Ebene parallel zur Ebene der Liegefläche 24 verläuft und die Führung von Luft im Raum zwischen der Zwischenfläche 28 und der Liegefläche 24 zur Temperierung der Liegefläche 24 erlaubt. Diese Ausführungsform der Erfindung trägt dem Erfordernis Rechnung, daß eine Temperierung der Liegefläche sowohl im Sinne einer Erwärmung bei kalter Liegefläche 24 als auch im Sinne einer Kühlung bei sich bei Betrieb der Strahler 25 erwärmender Liegefläche 24 erwünscht sein kann. Eine derartige Temperierung der Liegefläche 24 kann mittels Luft bewirkt werden, die eine für die Temperierung erforderliche Temperatur aufweist und durch den Raum zwischen der Zwischenfläche 28 und der Liegefläche 24 strömt.

Die Zwischenfläche 28 kann in dieser Ausführungsform der Erfindung aus demselben Material bestehen wie die Liegefläche 24. Aufgrund der wesentlich geringeren mechanischen Anforderungen, verglichen mit der Liegefläche 24 ― die Zwischenfläche 28 wird gewichtsmäßig so gut wie gar nicht belastet ― kann jedoch die Zwischenfläche aus einem viel weniger starken Material oder aus einem völlig anderen Material bestehen als die Liegefläche, vorausgesetzt, sie ist für zumindest bräunende UV-Strahlung hinreichend durchlässig. Eine 100 %ige Durchlässigkeit ist nicht erforderlich, eine möglichst hohe Durchlässigkeit für den bräunenden Anteil der UV-Strahlung ist jedoch erwünscht und angestrebt.

Der genaue Verlauf der Zwischenfläche 28, verglichen mit der Liegefläche 24, ist nicht von entscheidender Bedeutung, doch ist es bevorzugt, daß die Zwischenfläche 28 so angeordnet ist, daß sie in einer Ebene im wesentlichen parallel zur Ebene der Liegefläche verläuft. Dies ist deswegen vorteilhaft, weil sich dadurch ein Raum zwischen der Zwischenfläche 28 und der Liegefläche 24 ergibt, der annährend gleiche Dicke hat, keine Luftstaus beim Durchströmen der temperierten Luft ergibt und damit eine angenehme und gezielte Temperierung der Liegefläche 24 ermöglicht.

Die Temperierung der Liegefläche mittels der durch den Raum zwischen Liegefläche 24 und Zwischenfläche 28 strömender Luft kann während des Betriebs des Bräunungsgeräts 10 erfolgen, wobei es in diesem Fall häufig um eine Kühlung der Liegefläche 24 gegen die von den Strahlern 25 emittierte Wärme geht, oder kann vor und/oder nach dem Betrieb des Bräunungsgeräts 10 erfolgen, wobei je nach Wunsch entweder eine Erwärmung oder eine Kühlung oder auch eines nach dem anderen (z. B. erst Erwärmung, dann Kühlung) erfolgen kann. So kann in besonders vorteilhafter Weise eine jeder Situation angepaßte und auf den individuellen Wunsch des Benutzers abgestellte Temperierung des Bräunungsgeräts 10, insbesondere der Liegefläche 24, gegebenenfalls zusammen mit einer Temperierung des Bräunungsraums erfolgen.

Die Temperierung der zum Temperieren verwendeten Luft kann auf jeden denkbare und dem Fachmann an sich bekannte Weise geschehen. Insbesondere können für die Temperierung technische Mittel verwendet werden, wie sie im Stand der Technik beschrieben sind, beispielsweise in der Druckschrift DE-A 100 53 900.9. In besonders bevorzugten Ausführungsformen der Erfindung wird jedoch mittels eines separaten (vorzugsweise im Unterteil 21 des Bräunungsgeräts 10 untergebrachten) Lüfters die Luft in den Raum zwischen der Zwischenfläche 28 und der Liegefläche 24 eingespeist und die Liegefläche 24 mittels der durchströmenden (auf die gewünschte Temperatur gebrachten, also temperierten) Luft temperiert. Mit besonderem Vorteil ist dazu in dem erfindungsgemäßen Bräunungsgerät 10 der separate Lüfter mit einer (ebenfalls vorzugsweise im Unterteil 21 des Bräunungsgerät 10 untergebrachten) Kühl- oder Wärme-Einrichtung bzw. einem Klimagerät 30 verbunden. Vom Lüfter angeströmte Luft wird so in dem Klimagerät 30 temperiert und dann in den Raum zwischen der Zwischenfläche 28 und der Liegefläche 24 zur Temperierung der letzteren eingespeist. Dabei kann das Klimagerät 30 zum Erwärmen oder Kühlen der Luft Gebrauch von allen denkbaren Wärmequellen (z. B. den Strahlern 25) oder Wärmeverbrauchern (zur Erzeugung von "Kälte") machen, die im Stand der Technik für Erwärmungs- oder Kühlungszwecke, insbesondere bei Bräunungsgeräten, beschrieben sind.

Alternativ dazu oder in Ergänzung zu der vorher beschriebenen Ausführungsform entspricht es einer weiteren vorteilhaften Ausführungsform der Erfindung, wenn die die Liegefläche 24 temperierende Luft über eine Passage 20 vom Bräunungsraum 32 in den Raum zwischen der Zwischenfläche 28 und der Liegefläche 24 geführt ist. Damit wird die (beispielsweise kühlend) temperierende Luft des Bräunungsraums 32 anschließend auch für die Temperierung (z. B. Kühlung) der Liegefläche 24 genutzt, indem man sie aus dem Bräunungsraum 32 abzieht und über die Passage 20 unter der Liegefläche 24 hindurch strömen läßt. In einer besonders bevorzugten Ausführungsform geschieht dies in der Weise, daß die temperierende Luft vom Bräunungsraum 32 über eine kopfseitig und/oder fußseitig der Liegefläche 24 angeordnete Passage 20 in den Raum zwischen der Zwischenfläche 28 und der Liegefläche 24 geführt wird. Damit wird im wesentlichen eine vergleichbare Temperierung des Bräunungsraums 32 und der Liegefläche 24 erreicht, was für den Benutzer besonders angenehm ist.

Mit den vorbeschriebenen einzelnen Merkmalen oder einer Kombination beliebiger Merkmale der Erfindung, insbesondere den bevorzugten Merkmalen der Erfindung, wird eine für den Benutzer 1 angenehme Temperierung des Bräunungsraums 32 und gegebenenfalls auch der Liegefläche 24 erreicht. Die hierfür erforderlichen Einrichtungen befinden sich im wesentlichen im Unterteil 21 des Bräunungsgeräts 10 und belasten dadurch das Oberteil 31 kaum. Weiter wird eine zugfreie Temperierung des Körpers des Benutzers 1 im Bräunungsraum 32 ermöglicht, wodurch das Bräunungserlebnis intensiviert und auch ein Wahrnehmen akustischer Signale (z. B. Musik) nicht durch Luft-Strömungsgeräusche gestört wird.

In einer weiteren Ausführungsform der Erfindung umfaßt bei dem eingangs beschriebenen Bräunungsgerät 10 das Unterteil zwischen Liegefläche 24 und dem mindestens einen Satz Strahler 25 zur Abgabe von Strahlung mit zumindest einem Anteil an bräunender UV-Strahlung eine für zumindest bräunende UV-Strahlung durchlässige Zwischenfläche 28, die im wesentlichen in einer Ebene parallel zur Ebene der Liegefläche 24 verläuft und die Führung von Luft, insbesondere von temperierter Luft, im Raum zwischen der Zwischenfläche 28 und der Liegefläche 24 zur Temperierung der letzteren erlaubt.

Diese Ausführungsform der Erfindung trägt dem Erfordernis Rechnung, daß eine Temperierung der Liegefläche sowohl im Sinne einer Erwärmung bei kalter Liegefläche 24 als auch im Sinne einer Kühlung bei sich bei Betrieb der Strahler 25 erwärmender Liegefläche 24 erwünscht sein kann. Eine derartige Temperierung der Liegefläche 24 kann mittels Luft bewirkt werden, die eine für die Temperierung erforderliche Temperatur aufweist und durch den Raum zwischen der Zwischenfläche 28 und der Liegefläche 24 strömt.

Die Zwischenfläche 28 kann in dieser Ausführungsform der Erfindung aus demselben Material bestehen wie die Liegefläche 24. Aufgrund der wesentlich geringeren mechanischen Anforderungen, verglichen mit der Liegefläche 24 ― die Zwischenfläche 28 wird gewichtsmäßig so gut wie gar nicht belastet ― kann jedoch die Zwischenfläche aus einem viel weniger starken Material oder aus einem völlig anderen Material bestehen als die Liegefläche, vorausgesetzt, sie ist für zumindest bräunende UV-Strahlung hinreichend durchlässig. Eine 100 %ige Durchlässigkeit ist nicht erforderlich, eine möglichst hohe Durchlässigkeit für den bräunenden Anteil der UV-Strahlung ist jedoch erwünscht und angestrebt.

Der genaue Verlauf der Zwischenfläche 28, verglichen mit der Liegefläche 24, ist nicht von entscheidender Bedeutung, doch ist es bevorzugt, daß die Zwischenfläche 28 so angeordnet ist, daß sie in einer Ebene im wesentlichen parallel zur Ebene der Liegefläche verläuft. Dies ist deswegen vorteilhaft, weil sich dadurch ein Raum zwischen der Zwischenfläche 28 und der Liegefläche 24 ergibt, der annährend gleiche Dicke hat, keine Luftstaus beim Durchströmen der temperierten Luft ergibt und damit eine angenehme und gezielte Temperierung der Liegefläche 24 ermöglicht.

Die Temperierung der Liegefläche mittels der durch den Raum zwischen Liegefläche 24 und Zwischenfläche 28 strömender Luft kann während des Betriebs des Bräunungsgeräts 10 erfolgen, wobei es in diesem Fall häufig um eine Kühlung der Liegefläche 24 gegen die von den Strahlern 25 emittierte Wärme geht, oder kann vor und/oder nach dem Betrieb des Bräunungsgeräts 10 erfolgen, wobei je nach Wunsch entweder eine Erwärmung oder eine Kühlung oder auch eines nach dem anderen (z. B. erst Erwärmung, dann Kühlung) erfolgen kann. So kann in besonders vorteilhafter Weise eine jeder Situation angepaßte und auf den individuellen Wunsch des Benutzers abgestellte Temperierung des Bräunungsgeräts 10, insbesondere der Liegefläche 24, gegebenenfalls zusammen mit einer Temperierung des Bräunungsraums erfolgen.

Die Temperierung der zum Temperieren verwendeten Luft kann auf jeden denkbare und dem Fachmann an sich bekannte Weise geschehen. Insbesondere können für die Temperierung technische Mittel verwendet werden, wie sie im Stand der Technik beschrieben sind, beispielsweise in der Druckschrift DE-A 100 53 900.9. In besonders bevorzugten Ausführungsformen der Erfindung wird jedoch mittels eines separaten (vorzugsweise im Unterteil 21 des Bräunungsgeräts 10 untergebrachten) Lüfters die Luft in den Raum zwischen der Zwischenfläche 28 und der Liegefläche 24 eingespeist und die Liegefläche 24 mittels der durchströmenden (auf die gewünschte Temperatur gebrachten, also temperierten) Luft temperiert. Mit besonderem Vorteil ist dazu in dem erfindungsgemäßen Bräunungsgerät 10 der separate Lüfter mit einer (ebenfalls vorzugsweise im Unterteil 21 des Bräunungsgerät 10 untergebrachten) Kühl- oder Wärme-Einrichtung bzw. einem Klimagerät 30 verbunden. Vom Lüfter angeströmte Luft wird so in dem Klimagerät 30 temperiert und dann in den Raum zwischen der Zwischenfläche 28 und der Liegefläche 24 zur Temperierung der letzteren eingespeist. Dabei kann das Klimagerät 30 zum Erwärmen oder Kühlen der Luft Gebrauch von allen denkbaren Wärmequellen (z. B. den Strahlern 25) oder Wärmeverbrauchern (zur Erzeugung von "Kälte") machen, die im Stand der Technik für Erwärmungs- oder Kühlungszwecke, insbesondere bei Bräunungsgeräten, beschrieben sind.

Alternativ dazu oder in Ergänzung zu der vorher beschriebenen Ausführungsform entspricht es einer weiteren vorteilhaften Ausführungsform der Erfindung, wenn die die Liegefläche 24 temperierende Luft über eine Passage (20) vom Bräunungsraum 32 in den Raum zwischen der Zwischenfläche 28 und der Liegefläche 24 geführt ist. Damit wird (beispielsweise kühlend) temperierende Luft des Bräunungsraums 32 anschließend auch für die Temperierung (z. B. Kühlung) der Liegefläche 24 genutzt, indem man sie aus dem Bräunungsraum 32 abzieht und über die Passage (20) unter der Liegefläche 24 hindurch strömen läßt. In einer besonders bevorzugten Ausführungsform geschieht dies in der Weise, daß die temperierende Luft vom Bräunungsraum 32 über eine kopfseitig und/oder fußseitig der Liegefläche 24 angeordnete Passage 20 in den Raum zwischen der Zwischenfläche 28 und der Liegefläche 24 geführt wird. Damit wird im wesentlichen eine vergleichbare Temperierung des Bräunungsraums 32 und der Liegefläche 24 erreicht, was für den Benutzer besonders angenehm ist.

In einer weiteren bevorzugten Ausführungsform der vorstehend beschriebenen Erfindung weist das Unterteil 21 entlang zumindest eines Teils der Längsseiten 22a, 22b der Liegefläche 24 Düsen oder Kanäle 26 auf, durch die Luft ausströmen kann, wobei die Ausströmer-Düsen 26 und/oder Ausströmer-Kanäle 26 im wesentlichen in Richtung auf die Schutzfläche 34 des Oberteils 31 des Bräunungsgeräts gerichtet sind und in den Bräunungsraum 32 des Bräunungsgeräts 10 einen Luftstrom einspeisen, der nach Durchströmen des Bräunungsraums 34 den Körper des Benutzers 1 temperiert.

Mit im Unterteil 21 angeordneten Gebläse-Einrichtungen (nicht gezeigt) und Temperier-Einrichtungen (nicht gezeigt) über geeignete Leitungen verbundene Ausströmer-Düsen 26 oder Ausströmer-Kanäle 26 sind entlang zumindest eines Teils der Längsseiten 22a, 22b der Liegefläche 24, also vorzugsweise beidseitig der Liegefläche 24, angeordnet. Sie können aus einzelnen Düsen 26, also im wesentlichen runden oder ovalen Öffnungen, oder aus länglichen Kanälen 26 bestehen. Diese sind entweder konzentriert an bestimmten Stellen entlang der Längsseiten 22a, 22b der Liegefläche 24 angeordnet, um beispielsweise den Bräunungsraum 32 in Höhe bestimmter Stellen des Körpers des Benutzers 1, beispielsweise in Höhe des Gesichts, stärker zu temperieren, oder sind gleichmäßig entlang einer der beiden (oder auch entlang beiden) Längsseiten 22a, 22b der Liegefläche 24 angeordnet, um eine gleichmäßige, keinen Zug erzeugende Temperierung des Bräunungsraums 32 zu erreichen. Es ist erfindungsgemäß besonders bevorzugt, daß die Ausströmer-Düsen 26 oder Ausströmer-Kanäle 26 gleichmäßig entlang der beiden Längsseiten 22a, 22b der Liegefläche angeordnet sind. Es ist erfindungsgemäß möglich, daß sich entlang der Längsseiten 22a, 22b der Liegefläche sowohl Ausströmer-Düsen als auch Ausströmer-Kanäle oder auch nur Ausströmer-Düsen 26 oder auch nur Ausströmer-Kanäle 26 finden, wobei die Reihenfolge und Anordnung vom Fachmann im wesentlichen frei und entsprechend den apparativen Erfordernissen frei gewählt werden kann. In einer weiteren bevorzugten Ausführungsform ist es auch möglich, daß entlang beider Längsseiten 22a, 22b der Liegefläche 24 über im wesentlichen die gesamte Länge auf jeder Seite der Liegefläche 24 je ein Ausströmer-Kanal 26 angeordnet ist.

Die Ausströmer-Düsen oder ―Kanäle 26 speisen in den Bräunungsraum 32 des Bräunungsgeräts 10 einen temperierten Luftstrom ein, beispielsweise einen kühlen Luftstrom. Dieser ist im wesentlichen in Richtung auf die Schutzfläche 34 der Oberteils 31 des Bräunungsgeräts 10 gerichtet. Hierunter wird verstanden, daß der Luftstrom nicht - wie in herkömmlichen Bräunungsgeräten ― direkt auf den Körper des Benutzers gerichtet ist und eine direkte, oft als unangenehm empfundene und mitunter von störenden Luft-Strömungsgeräuschen begleitete Temperierung, beispielsweise eine Kühlung des Körpers des Benutzers 1 bewirkt. Vielmehr strömt der Luftstrom in den Bräunungsraum 32 in Richtung auf dessen obere Begrenzung durch die Schutzfläche 34 des Oberteils 31 und erreicht den Körper des Benutzers 1 erst nach Durchströmen des Bräunungsraums 32. So wird überraschenderweise nicht nur das störende Luft-Strömungsgeräusch (beispielsweise an den Ohren des Benutzers 1) vermieden, sondern auch eine als sanft und angenehm empfundene Kühlung bewirkt, die vom Benutzer 1 nicht als Zug erfahren wird.

In einer weiter bevorzugten Ausführungsform der Erfindung ist die für zumindest bräunende UV-Strahlung durchlässige Schutzfläche 34 des Oberteils 31 zur Bildung des in Längsrichtung des Bräunungsgeräts 10 im wesentlichen tunnelförmigen Bräunungsraums 32 aus Sicht des Benutzers konkav gewölbt. Die konkav gewölbte Schutzfläche ermöglicht in vorteilhafter Weise, daß der aus den Ausströmer-Düsen oder ―Kanälen 26 austretende Luftstrom entlang der konkaven Wölbung der Schutzfläche 34 strömen kann und dadurch gleichmäßig und gezielt wieder in den Bräunungsraum 32 zurückgeführt wird.

Erfindungsgemäß ist es weiter bevorzugt, daß die für zumindest bräunende UV-Strahlung durchlässige Schutzfläche 34 des Oberteils 31 des Bräunungsgeräts 10 im Scheitel 36 der konkaven Wölbung in Längsrichtung des Oberteils 31 einen im Querschnitt keilförmigen Luft-Umlenker 38 aufweist. Der Luft-Umlenker 38 ist weiter bevorzugt aus einem Material hergestellt, das ― wie die Schutzfläche 34 für den Benutzer 1 ― für zumindest bräunende UV-Strahlung durchlässig ist, um zu verhindern, daß diese Einrichtung 38 einen Teil der von den Strahlern 35 emittierten bräunenden UV-Strahlung absorbiert oder abschirmt. Der Luft-Umlenker 38 ist von besonderem Vorteil deswegen, weil sich die durch die Ausströmer-Düsen und/oder ―Kanäle 26 in den Bräunungsraum 32 eingespeiste Luft mit seiner Hilfe von der konkaven Wölbung der Schutzfläche 34 auf den Benutzer lenken läßt. In einer besonders bevorzugten Ausführungsform wird sogar die auf beiden Längsseiten 22a, 22b seitlich der Liegefläche durch die Ausströmer-Düsen und/oder ―Kanäle 26 einströmende Luft mittels des Luft-Umlenkers 38 in der Mitte des Bräunungsraums 32 verwirbelt, was zu einem als besonders angenehm empfundenen temperierenden Luftstrom am Körper des Benutzers 1 führt.

In einer noch weiter bevorzugten, mit besonderem Vorteil verwirklichten Ausführungsform der Erfindung ist der Luft-Umlenker 38 Teil der für zumindest bräunende UV-Strahlung durchlässigen Schutzfläche 34. In diesem Fall ist natürlich der Luft-Umlenker 38 aus demselben Material wie die Schutzfläche 34 und ist damit auch hinsichtlich seiner Durchlässigkeit für zumindest bräunende UV-Strahlung mit der Durchlässigkeit der Schutzfläche 34 identisch. Diese Ausführungsform ist auch aus Herstellungs-Sicht besonders vorteilhaft, da die Schutzfläche 34 zusammen mit dem Luft-Umlenker 38 integral gestaltet ist und entsprechend in einem Schritt hergestellt werden kann.

In einer dazu alternativen Ausführungsform kann der Luft-Umlenker 38 auf die für zumindest bräunende UV-Strahlung durchlässige Schutzfläche 34 aufgebracht werden, was natürlich auch nachträglich geschehen kann. Damit wird ein Nachrüsten der Schutzfläche 34 des Oberteils 32 des Bräunungsgeräts 10 mit dem Luft-Umlenker 38 möglich, so daß auch bereits laufenden Geräte die Vorteile der Lenkung des Luftstroms an der Schutzfläche 34 verliehen werden können. Die Aufbringung des Luft-Umlenkers 38 auf die Schutzfläche 34 kann auf jede beliebige, dem Fachmann bekannte Art und Weise erfolgen. Ganz besonders bevorzugt ist jedoch ein Aufkleben des Luft-Umlenkers 38 auf die Schutzfläche 34 des Oberteils 31 des Bräunungsgeräts 10.

Der Luft-Umlenker 38 auf der (oder integriert in die) für zumindest bräunende UV-Strahlung durchlässige Schutzfläche 34 kann eine völlig glatte Oberfläche aufweisen. Erfindungsgemäß ist es jedoch noch weiter bevorzugt, daß der Luft-Umlenker 38 eine der Verteilung und/oder Verwirbelung der strömenden Luft dienende Oberflächenstruktur 39 aufweist. Eine solche Oberflächenstruktur 39 kann zu einer noch besseren Verteilung des Luftstroms oder gar zu dessen Verwirbelung im Bräunungsraum 32 des Oberteils 31 des Bräunungsgeräts 10 führen, und die Temperierung des Bräunungsraums und des Körpers des Benutzers 1 erfolgen auf diesem Weg noch nachhaltiger und angenehmer. Die Oberflächenstruktur kann von beliebiger Art sein, wie sie der Fachmann für die angegebenen Zwecke kennt, ohne daß die Erfindung diesbezüglich irgendwelchen Beschränkungen unterliegt. In einer mit besonderem Vorteil verwirklichten Ausführungsform weist der Luft-Umlenker 38 Vertiefungen, Spiralen und/oder Wellen auf, die der Verteilung und/oder Verwirbelung der strömenden Luft dienen.

In einer alternativen oder auch zusammen mit der vorstehend beschriebenen Ausführungsform gemeinsam verwirklichten vorteilhaften Ausführungsform der Erfindung weist der Luft-Umlenker 38 Einrichtungen 40 auf, mit deren Hilfe die strömende Luft separat auf den Körper oder separat auf das Gesicht des Benutzers 1 geleitet werden kann. Solche Einrichtungen 40 sind dem Fachmann ebenfalls bekannt und können entweder statisch oder individuell einstellbar sein, damit in vorteilhafter Weise der Luftstrom auf die Stellen des Körpers oder des Gesichts gelenkt werden kann, die dem Benutzer 1 am angenehmsten sind.

Mit den vorbeschriebenen einzelnen Merkmalen oder einer Kombination beliebiger Merkmale der Erfindung, insbesondere den bevorzugten Merkmalen der Erfindung, wird eine für den Benutzer 1 angenehme Temperierung des Bräunungsraums 32 und gegebenenfalls auch der Liegefläche 24 erreicht. Die hierfür erforderlichen Einrichtungen befinden sich im wesentlichen im Unterteil 21 des Bräunungsgeräts 10 und belasten dadurch das Oberteil 31 kaum. Weiter wird eine zugfreie Temperierung des Körpers des Benutzers 1 im Bräunungsraum 32 ermöglicht, wodurch das Bräunungserlebnis intensiviert und auch ein Wahrnehmen akustischer Signale (z. B. Musik) nicht durch Luft-Strömungsgeräusche gestört wird.

In einer weiteren bevorzugten Ausführungsform weist das erfindungsgemäße Bräunungsgerät 10 ein Unterteil 21 auf, bei dem entlang zumindest eines Teils der Längsseiten 22a, 22b der Liegefläche 24 Ausströmer-Düsen 26 und/oder Ausströmer-Kanäle 26 angeordnet sind, durch die Luft ausströmen kann, wobei die Ausströmer-Düsen 26 und/oder Ausströmer-Kanäle 26 im wesentlichen in Richtung auf die Schutzfläche 34 des Oberteils 31 des Bräunungsgeräts gerichtet sind und in den Bräunungsraum 32 des Bräunungsgeräts 10 einen Luftstrom einspeisen, der den Bräunungsraum 32 auch bei nicht arbeitenden Strahlern 25, 35 temperiert. Dieses Konzept eines quasi "geschlossenen" Bräunungsraums 32 ermöglicht es, den Bräunungsraum 32 auch dann zu temperieren, wenn das Bräunungsgerät 10 (noch) nicht in Betrieb ist. Dies kann beispielsweise von Nutzen sein, wenn das Bräunungsgerät 10 längere Zeit ausgeschaltet war und in einem kühlen oder kühl klimatisierten Raum steht oder wenn das Bräunungsgerät 10 längere Zeit unter Emission von Wärme in Betrieb war, aufgeheizt ist und für den nächsten Benutzer gekühlt werden soll oder wenn das Bräunungsgerät 10 in einem warmen, nicht klimatisierten Raum steht und für eine Benutzung durch den nächsten Benutzer kühl temperiert werden soll.

Mit im Unterteil 21 angeordneten Gebläse-Einrichtungen (nicht gezeigt) und Temperier-Einrichtungen (nicht gezeigt) über geeignete Leitungen verbundene Ausströmer-Düsen 26 oder Ausströmer-Kanäle 26 sind entlang zumindest eines Teils der Längsseiten 22a, 22b der Liegefläche 24, also vorzugsweise beidseitig der Liegefläche 24, angeordnet. Sie können aus einzelnen Düsen 26, also im wesentlichen runden oder ovalen Öffnungen, oder aus länglichen Kanälen 26 bestehen. Diese sind entweder konzentriert an bestimmten Stellen entlang der Längsseiten 22a, 22b der Liegefläche 24 angeordnet oder sind gleichmäßig entlang einer der beiden (oder auch entlang beiden) Längsseiten 22a, 22b der Liegefläche 24 angeordnet. Für die Anordnung entscheidendes Kriterium ist die gleichmäßige Temperierung des Bräunungsraums 32 auch bei nicht arbeitenden Strahlern 25, 35. Es ist erfindungsgemäß besonders bevorzugt, daß die Ausströmer-Düsen 26 oder Ausströmer-Kanäle 26 gleichmäßig entlang der beiden Längsseiten 22a, 22b der Liegefläche angeordnet sind. Es ist erfindungsgemäß möglich, daß sich entlang der Längsseiten 22a, 22b der Liegefläche sowohl Ausströmer-Düsen als auch Ausströmer-Kanäle oder auch nur Ausströmer-Düsen 26 oder auch nur Ausströmer-Kanäle 26 finden, wobei die Reihenfolge und Anordnung vom Fachmann im wesentlichen frei und entsprechend den apparativen Erfordernissen frei gewählt werden kann. In einer weiteren bevorzugten Ausführungsform ist es auch möglich, daß entlang beider Längsseiten 22a, 22b der Liegefläche 24 über im wesentlichen die gesamte Länge auf jeder Seite der Liegefläche 24 je ein Ausströmer-Kanal 26 angeordnet ist.

Die Ausströmer-Düsen oder ―Kanäle 26 speisen in den Bräunungsraum 32 des Bräunungsgeräts 10 einen temperierten Luftstrom ein, beispielsweise einen kühlen Luftstrom. Dieser ist im wesentlichen in Richtung auf die Schutzfläche 34 der Oberteils 31 des Bräunungsgeräts 10 gerichtet. Hierunter wird verstanden, daß der Luftstrom in den Bräunungsraum 32 in Richtung auf dessen obere Begrenzung durch die Schutzfläche 34 des Oberteils 31 gerichtet ist.

In einer noch mehr bevorzugten Ausführungsform der Erfindung wird der über die Ausströmer-Düsen oder ―Kanäle 26 eingespeiste Luftstrom nach Durchströmen des Bräunungsgeräts 10 kopfseitig oder fußseitig der Liegefläche 24 abgesaugt und einem temperierenden (gegebenenfalls mit einem Lüfter gekoppelten) Klimagerät 30 zugeführt, das den temperierenden Luftstrom wieder in den Bräunungsraum einspeist. Auf diese Weise wird ein energiesparender und ökonomischer Kreislauf der temperierenden Luft eingerichtet, mit dem die Temperierung des Bräunungsraums 32 einfach erreicht werden kann.

Der Ökonomisierung dieses Kreislaufs dient eine weitere bevorzugte Ausführungsform der Erfindung, gemäß der die Kopf- und/oder die Fußseite des Bräunungsraums 32 des Bräunungsgeräts 10 jeweils eine verschließbare Wand 29a, 29b zum Verschließen des Bräunungsraums 32 aufweisen. Unter "verschließbar" und "Verschließen" wird im Rahmen dieses Merkmals der Erfindung in der Beschreibung und in den Patentansprüchen kein hermetischen Verschließen eines von Luft gefüllten Volumenraums verstanden, was sich bei einem Bräunungsgerät 10 nur unter schwierigen technischen Bedingungen erreichen ließe, sondern eine weitgehende Abschirmung des Bräunungsraums 32 gegenüber von außen eindringender, nicht im Kreislauf Ausströmer-Düsen und/oder ―Kanäle 26 → Bräunungsraum 32 → kopf- und/oder fußseitige Absaugung → Lüfter und/oder Klimagerät → Ausströmer-Düsen und/oder ―Kanäle 26 geführter Luft zur Vermeidung von Wärme- oder Kälte-Verlusten. Die verschließbaren Wände 29a, 29b können jedes beliebige Material umfassen und bestehen insbesondere aus einem transparenten, leichten und gegen UV-Strahlung unempfindlichen Material. Besonders bevorzugt wird ein aus Kunststoff, insbesondere Polymethylmethacrylat (PMMA), und Glas gewähltes Material verwendet.

In einer weiter bevorzugten Ausführungsform der Erfindung ist die für zumindest bräunende UV-Strahlung durchlässige Schutzfläche 34 des Oberteils 31 zur Bildung des in Längsrichtung des Bräunungsgeräts 10 im wesentlichen tunnelförmigen Bräunungsraums 32 aus Sicht des Benutzers konkav gewölbt. Die konkav gewölbte Schutzfläche ermöglicht in vorteilhafter Weise, daß der aus den Ausströmer-Düsen oder ―Kanälen 26 austretende Luftstrom entlang der konkaven Wölbung der Schutzfläche 34 strömen kann und dadurch gleichmäßig und gezielt wieder in den Bräunungsraum 32 zurückgeführt wird.

Erfindungsgemäß ist es weiter bevorzugt, daß die für zumindest bräunende UV-Strahlung durchlässige Schutzfläche 34 des Oberteils 31 des Bräunungsgeräts 10 im Scheitel 36 der konkaven Wölbung in Längsrichtung des Oberteils 31 einen im Querschnitt keilförmigen Luft-Umlenker 38 aufweist. Der Luft-Umlenker 38 ist weiter bevorzugt aus einem Material hergestellt, das ― wie die Schutzfläche 34 für den Benutzer 1 ― für zumindest bräunende UV-Strahlung durchlässig ist, um zu verhindern, daß diese Einrichtung 38 einen Teil der von den Strahlern 35 emittierten bräunenden UV-Strahlung absorbiert oder abschirmt. Der Luft-Umlenker 38 ist von besonderem Vorteil deswegen, weil sich die durch die Ausströmer-Düsen und/oder ―Kanäle 26 in den Bräunungsraum 32 eingespeiste Luft mit seiner Hilfe von der konkaven Wölbung der Schutzfläche 34 auf den Benutzer lenken läßt. In einer besonders bevorzugten Ausführungsform wird sogar die auf beiden Längsseiten 22a, 22b seitlich der Liegefläche durch die Ausströmer-Düsen und/oder -Kanäle 26 einströmende Luft mittels des Luft-Umlenkers 38 in der Mitte des Bräunungsraums 32 verwirbelt, was zu einem als besonders angenehm empfundenen temperierenden Luftstrom am Körper des Benutzers 1 führt.

In einer noch weiter bevorzugten, mit besonderem Vorteil verwirklichten Ausführungsform der Erfindung ist der Luft-Umlenker 38 Teil der für zumindest bräunende UV-Strahlung durchlässigen Schutzfläche 34. In diesem Fall ist natürlich der Luft-Umlenker 38 aus demselben Material wie die Schutzfläche 34 und ist damit auch hinsichtlich seiner Durchlässigkeit für zumindest bräunende UV-Strahlung mit der Durchlässigkeit der Schutzfläche 34 identisch. Diese Ausführungsform ist auch aus Herstellungs-Sicht besonders vorteilhaft, da die Schutzfläche 34 zusammen mit dem Luft-Umlenker 38 integral gestaltet ist und entsprechend in einem Schritt hergestellt werden kann.

In einer dazu alternativen Ausführungsform kann der Luft-Umlenker 38 auf die für zumindest bräunende UV-Strahlung durchlässige Schutzfläche 34 aufgebracht werden, was natürlich auch nachträglich geschehen kann. Damit wird ein Nachrüsten der Schutzfläche 34 des Oberteils 32 des Bräunungsgeräts 10 mit dem Luft-Umlenker 38 möglich, so daß auch bereits laufenden Geräte die Vorteile der Lenkung des Luftstroms an der Schutzfläche 34 verliehen werden können. Die Aufbringung des Luft-Umlenkers 38 auf die Schutzfläche 34 kann auf jede beliebige, dem Fachmann bekannte Art und Weise erfolgen. Ganz besonders bevorzugt ist jedoch ein Aufkleben des Luft-Umlenkers 38 auf die Schutzfläche 34 des Oberteils 31 des Bräunungsgeräts 10.

Der Luft-Umlenker 38 auf der (oder integriert in die) für zumindest bräunende UV-Strahlung durchlässige Schutzfläche 34 kann eine völlig glatte Oberfläche aufweisen. Erfindungsgemäß ist es jedoch noch weiter bevorzugt, daß der Luft-Umlenker 38 eine der Verteilung und/oder Verwirbelung der strömenden Luft dienende Oberflächenstruktur 39 aufweist. Eine solche Oberflächenstruktur 39 kann zu einer noch besseren Verteilung des Luftstroms oder gar zu dessen Verwirbelung im Bräunungsraum 32 des Oberteils 31 des Bräunungsgeräts 10 führen, und die Temperierung des Bräunungsraums und des Körpers des Benutzers 1 erfolgen auf diesem Weg noch nachhaltiger und angenehmer. Die Oberflächenstruktur kann von beliebiger Art sein, wie sie der Fachmann für die angegebenen Zwecke kennt, ohne daß die Erfindung diesbezüglich irgendwelchen Beschränkungen unterliegt. In einer mit besonderem Vorteil verwirklichten Ausführungsform weist der Luft-Umlenker 38 Vertiefungen, Spiralen und/oder Wellen auf, die der Verteilung und/oder Verwirbelung der strömenden Luft dienen.

In einer alternativen oder auch zusammen mit der vorstehend beschriebenen Ausführungsform gemeinsam verwirklichten vorteilhaften Ausführungsform der Erfindung weist der Luft-Umlenker 38 Einrichtungen 40 auf, mit deren Hilfe die strömende Luft separat auf den Körper oder separat auf das Gesicht des Benutzers 1 geleitet werden kann. Solche Einrichtungen 40 sind dem Fachmann ebenfalls bekannt und können entweder statisch oder individuell einstellbar sein, damit in vorteilhafter Weise der Luftstrom auf die Stellen des Körpers oder des Gesichts gelenkt werden kann, die dem Benutzer 1 am angenehmsten sind.

In einer weiteren vorteilhaften Ausführungsform der Erfindung, die zusammen mit den vorstehenden Merkmalen des Bräunungsgeräts verwirklicht werden kann, weist das Unterteil 21 des Bräunungsgeräts 10 zwischen Liegefläche 24 und dem mindestens einen Satz Strahler 25 zur Abgabe von Strahlung mit zumindest einem Anteil an bräunender UV-Strahlung eine für zumindest bräunende UV-Strahlung durchlässige Zwischenfläche 28 auf, die im wesentlichen in einer Ebene parallel zur Ebene der Liegefläche 24 verläuft und die Führung von Luft im Raum zwischen der Zwischenfläche 28 und der Liegefläche 24 zur Temperierung der Liegefläche 24 erlaubt. Diese Ausführungsform der Erfindung trägt dem Erfordernis Rechnung, daß eine Temperierung der Liegefläche sowohl im Sinne einer Erwärmung bei kalter Liegefläche 24 als auch im Sinne einer Kühlung bei sich bei Betrieb der Strahler 25 erwärmender Liegefläche 24 erwünscht sein kann. Eine derartige Temperierung der Liegefläche 24 kann mittels Luft bewirkt werden, die eine für die Temperierung erforderliche Temperatur aufweist und durch den Raum zwischen der Zwischenfläche 28 und der Liegefläche 24 strömt.

Die Zwischenfläche 28 kann in dieser Ausführungsform der Erfindung aus demselben Material bestehen wie die Liegefläche 24. Aufgrund der wesentlich geringeren mechanischen Anforderungen, verglichen mit der Liegefläche 24 ― die Zwischenfläche 28 wird gewichtsmäßig so gut wie gar nicht belastet ― kann jedoch die Zwischenfläche aus einem viel weniger starken Material oder aus einem völlig anderen Material bestehen als die Liegefläche, vorausgesetzt, sie ist für zumindest bräunende UV-Strahlung hinreichend durchlässig. Eine 100 %ige Durchlässigkeit ist nicht erforderlich, eine möglichst hohe Durchlässigkeit für den bräunenden Anteil der UV-Strahlung ist jedoch erwünscht und angestrebt.

Der genaue Verlauf der Zwischenfläche 28, verglichen mit der Liegefläche 24, ist nicht von entscheidender Bedeutung, doch ist es bevorzugt, daß die Zwischenfläche 28 so angeordnet ist, daß sie in einer Ebene im wesentlichen parallel zur Ebene der Liegefläche verläuft. Dies ist deswegen vorteilhaft, weil sich dadurch ein Raum zwischen der Zwischenfläche 28 und der Liegefläche 24 ergibt, der annährend gleiche Dicke hat, keine Luftstaus beim Durchströmen der temperierten Luft ergibt und damit eine angenehme und gezielte Temperierung der Liegefläche 24 ermöglicht.

Die Temperierung der Liegefläche mittels der durch den Raum zwischen Liegefläche 24 und Zwischenfläche 28 strömender Luft kann während des Betriebs des Bräunungsgeräts 10 erfolgen, wobei es in diesem Fall häufig um eine Kühlung der Liegefläche 24 gegen die von den Strahlern 25 emittierte Wärme geht, oder kann vor und/oder nach dem Betrieb des Bräunungsgeräts 10 erfolgen, wobei je nach Wunsch entweder eine Erwärmung oder eine Kühlung oder auch eines nach dem anderen (z. B. erst Erwärmung, dann Kühlung) erfolgen kann. So kann in besonders vorteilhafter Weise eine jeder Situation angepaßte und auf den individuellen Wunsch des Benutzers abgestellte Temperierung des Bräunungsgeräts 10, insbesondere der Liegefläche 24, gegebenenfalls zusammen mit einer Temperierung des Bräunungsraums erfolgen.

Die Temperierung der zum Temperieren verwendeten Luft kann auf jeden denkbare und dem Fachmann an sich bekannte Weise geschehen. Insbesondere können für die Temperierung technische Mittel verwendet werden, wie sie im Stand der Technik beschrieben sind, beispielsweise in der Druckschrift DE-A 100 53 900.9. In besonders bevorzugten Ausführungsformen der Erfindung wird jedoch mittels eines separaten (vorzugsweise im Unterteil 21 des Bräunungsgeräts 10 untergebrachten) Lüfters die Luft in den Raum zwischen der Zwischenfläche 28 und der Liegefläche 24 eingespeist und die Liegefläche 24 mittels der durchströmenden (auf die gewünschte Temperatur gebrachten, also temperierten) Luft temperiert. Mit besonderem Vorteil ist dazu in dem erfindungsgemäßen Bräunungsgerät 10 der separate Lüfter mit einer (ebenfalls vorzugsweise im Unterteil 21 des Bräunungsgerät 10 untergebrachten) Kühl- oder Wärme-Einrichtung bzw. einem Klimagerät 30 verbunden. Vom Lüfter angeströmte Luft wird so in dem Klimagerät 30 temperiert und dann in den Raum zwischen der Zwischenfläche 28 und der Liegefläche 24 zur Temperierung der letzteren eingespeist. Dabei kann das Klimagerät 30 zum Erwärmen oder Kühlen der Luft Gebrauch von allen denkbaren Wärmequellen (z. B. den Strahlern 25) oder Wärmeverbrauchern (zur Erzeugung von "Kälte") machen, die im Stand der Technik für Erwärmungs- oder Kühlungszwecke, insbesondere bei Bräunungsgeräten, beschrieben sind.

Alternativ dazu oder in Ergänzung zu der vorher beschriebenen Ausführungsform entspricht es einer weiteren vorteilhaften Ausführungsform der Erfindung, wenn die die Liegefläche 24 temperierende Luft über eine Passage 20 vom Bräunungsraum 32 in den Raum zwischen der Zwischenfläche 28 und der Liegefläche 24 geführt ist. Damit wird die (beispielsweise kühlend) temperierende Luft des Bräunungsraums 32 anschließend auch für die Temperierung (z. B. Kühlung) der Liegefläche 24 genutzt, indem man sie aus dem Bräunungsraum 32 abzieht und über die Passage 20 unter der Liegefläche 24 hindurch strömen läßt. In einer besonders bevorzugten Ausführungsform geschieht dies in der Weise, daß die temperierende Luft vom Bräunungsraum 32 über eine kopfseitig und/oder fußseitig der Liegefläche 24 angeordnete Passage 20 in den Raum zwischen der Zwischenfläche 28 und der Liegefläche 24 geführt wird. Damit wird im wesentlichen eine vergleichbare Temperierung des Bräunungsraums 32 und der Liegefläche 24 erreicht, was für den Benutzer besonders angenehm ist.

Mit den vorbeschriebenen einzelnen Merkmalen oder einer Kombination beliebiger Merkmale der Erfindung, insbesondere den bevorzugten Merkmalen der Erfindung, wird eine für den Benutzer 1 angenehme Temperierung des Bräunungsraums 32 und gegebenenfalls auch der Liegefläche 24 erreicht. Die hierfür erforderlichen Einrichtungen befinden sich im wesentlichen im Unterteil 21 des Bräunungsgeräts 10 und belasten dadurch das Oberteil 31 kaum. Weiter wird eine zugfreie Temperierung des Bräunungsraums 32 und damit indirekt auch des Körpers des Benutzers 1 im Bräunungsraum 32 ermöglicht, wodurch das Bräunungserlebnis intensiviert und auch ein Wahrnehmen akustischer Signale (z. B. Musik) nicht durch Luft-Strömungsgeräusche gestört wird. Die zuletzt beschriebene Ausführungsform des erfindungsgemäßen Bräunungsgeräts 10 ist zudem vorteilhaft dahingehend, daß der Bräunungsraum 32 mit geringem Aufwand temperiert werden kann, was sich in Ländern als günstig erweist, in denen hohe Außentemperaturen herrschen und die Bräunungsgeräte nicht in klimatisierten Räumen stehen. Durch die Zuführung und Absaugung der temperierenden Luft über Aggregate im Unterteil des Bräunungsgeräts 10 werden kurze, keine Wärme- oder "Kälte"-Verluste bedingende Kreisläufe geschaffen, die mit einfachen, zum Teil mechanischen Mitteln wie Klappen zu regeln sind, die gegebenenfalls mit einer Temperatursteuerung verbunden werden können.

Die Erfindung betrifft weiter ein Verfahren zum Temperieren eines Bräunungsraums 32 eines Bräunungsgeräts 10. Das Bräunungsgerät 10 kann ein übliches Bräunungsgerät sein, umfaßt jedoch in der bevorzugten Ausführungsform des Verfahrens
- ein standfestes Unterteil 21, enthaltend elektrische Aggregate, Schalt-Einrichtungen und Verbindungs-Einrichtungen; und
- ein entweder an einer der Längsseiten 22 des Unterteils 21 über Scharniere oder Gelenke 23 angebrachtes, über die Scharniere oder Gelenke 23 von dem Unterteil 21 weg und auf das Unterteil 21 hin zur Bildung eines im wesentlichen tunnelförmigen Bräunungsraumes 32 klappbares oder schwenkbares oder an einer separaten Aufhängung 33 von dem Unterteil 21 weg hebbares und auf das Unterteil 21 hin zur Bildung eines im wesentlichen tunnelförmigen Bräunungsraumes 32 senkbares Oberteil 31; wobei
- das Unterteil 21 eine für zumindest bräunende UV-Strahlung durchlässige Liegefläche 24 für den Benutzer 1 und unterhalb der Liegefläche 24 mindestens einen Satz in Längsrichtung des Bräunungsgeräts 10 angeordneter Strahler 25 zur Abgabe von Strahlung mit zumindest einem Anteil an bräunender UV-Strahlung in Richtung auf das Oberteil 31 und/oder zumindest einen Teil des Körpers des Benutzers 1 gerichtet mit den elektrischen Aggregaten, Schalt-Einrichtungen und/oder Verbindungs-Einrichtungen direkt oder indirekt verbunden aufweist; und
- das Oberteil 31 eine für zumindest bräunende UV-Strahlung durchlässige Schutzfläche 34 für den Benutzer 1 und oberhalb der Schutzfläche 34 mindestens einen Satz in Längsrichtung des Bräunungsgeräts 10 angeordneter Strahler 35 zur Abgabe von Strahlung mit zumindest einem Anteil an bräunender UV-Strahlung in Richtung auf das Unterteil 21 und/oder zumindest einen Teil des Körpers des Benutzers 1 gerichtet aufweist.

Im Rahmen des erfindungsgemäßen Verfahrens speist man über entlang zumindest eines Teils der Längsseiten 22a, 22b der Liegefläche 24 des Unterteils 21 angeordnete Ausströmer-Düsen 26 oder Ausströmer-Kanäle 26, die im wesentlichen in Richtung auf die Schutzfläche 34 des Oberteils 31 gerichtet sind, Luft und bevorzugt einen temperierten Luftstrom in den Bräunungsraum 32 ein, die/der den Bräunungsraum 32 bei Durchströmen temperiert.

In einem weiteren Verfahren gemäß der Erfindung, das sich auf das wie vorstehend im einzelnen beschriebene Bräunungsgerät 10 bezieht und dem Temperieren des Körpers eines Benutzers 1 des Bräunungsgeräts 10 dient, speist man mittels entlang zumindest der Längsseiten 22a, 22b der Liegefläche 24 des Unterteils 21 des Bräunungsgeräts 10 angeordneter Ausströmer-Düsen 26 oder Ausströmer-Kanäle 26 im wesentlichen in Richtung auf die Schutzfläche 34 des Oberteils 31 des Bräunungsgeräts 10 in den Bräunungsraum 32 einen Luftstrom ein, der den Körper des Benutzers 1 nach Durchströmen des Bräunungsraums 32 temperiert.

In einer vorteilhaften Ausführungsform des erfindungsgemäßen Verfahrens sieht man an dem Unterteil 21 des Bräunungsgeräts 10 entlang zumindest eines Teils der Längsseiten 22a, 22b der Liegefläche 24 Düsen oder Kanäle 26 vor, durch die Luft ausströmen kann, wobei man die Ausströmer-Düsen 26 und/oder Ausströmer-Kanäle 26 im wesentlichen in Richtung auf die Schutzfläche 34 des Oberteils 31 des Bräunungsgeräts richtet. So speist man in den Bräunungsraum 32 des Bräunungsgeräts 10 einen Luftstrom ein, der den Bräunungsraum 32 bei Durchströmen temperiert, wenn sich kein Benutzer 1 in dem Bräunungsraum 32 aufhält, oder der nach Durchströmen des Bräunungsraums 34 den Körper des Benutzers 1 temperiert, wenn sich ein Benutzer 1 in dem Bräunungsraum 32 aufhält.

Man ordnet erfindungsgemäß mit im Unterteil 21 angeordneten Gebläse-Einrichtungen (nicht gezeigt) und Temperier-Einrichtungen (nicht gezeigt) über geeignete Leitungen verbundene Ausströmer-Düsen 26 oder Ausströmer-Kanäle 26 entlang zumindest eines Teils der Längsseiten 22a, 22b der Liegefläche 24, also vorzugsweise beidseitig der Liegefläche 24, an. Die Ausströmer-Düsen und/oder ―Kanäle 26 können aus einzelnen Düsen 26, also im wesentlichen runden oder ovalen Öffnungen, oder aus länglichen Kanälen 26 bestehen. Diese ordnet man entweder konzentriert an bestimmten Stellen entlang der Längsseiten 22a, 22b der Liegefläche 24 an, um beispielsweise bestimmte Stellen des Körpers des Benutzers 1, beispielsweise das Gesicht, stärker zu temperieren, oder man ordnet sie gleichmäßig entlang einer der beiden (oder auch entlang beiden) Längsseiten 22a, 22b der Liegefläche 24 angeordnet, um eine gleichmäßige, keinen Zug erzeugende Temperierung zu erreichen. Es ist erfindungsgemäß besonders bevorzugt, die Ausströmer-Düsen 26 oder Ausströmer-Kanäle 26 gleichmäßig entlang der beiden Längsseiten 22a, 22b der Liegefläche anzuordnen. Es ist erfindungsgemäß möglich, daß man entlang der Längsseiten 22a, 22b der Liegefläche sowohl Ausströmer-Düsen als auch Ausströmer-Kanäle oder auch nur Ausströmer-Düsen 26 oder auch nur Ausströmer-Kanäle 26 vorsieht, wobei die Reihenfolge und Anordnung vom Fachmann im wesentlichen frei und entsprechend den apparativen Erfordernissen frei gewählt werden kann. In einer weiteren bevorzugten Ausführungsform ist es auch möglich, entlang beider Längsseiten 22a, 22b der Liegefläche 24 über im wesentlichen die gesamte Länge auf jeder Seite der Liegefläche 24 je einen Ausströmer-Kanal 26 vorzusehen.

Mittels der Ausströmer-Düsen oder ―Kanäle 26 speist man in den Bräunungsraum 32 des Bräunungsgeräts 10 einen temperierten Luftstrom ein, beispielsweise einen kühlen Luftstrom. Diesen richtet man im wesentlichen in Richtung auf die Schutzfläche 34 der Oberteils 31 des Bräunungsgeräts 10. Hierunter wird verstanden, daß man den Luftstrom nicht - wie in herkömmlichen Bräunungsgeräten ― direkt auf den Körper des Benutzers richtet und eine direkte, oft als unangenehm empfundene und mitunter von störenden Luft-Strömungsgeräuschen begleitete Temperierung bzw. Kühlung des Körpers des Benutzers 1 bewirkt. Vielmehr speist man den Luftstrom in den Bräunungsraum 32 in Richtung auf dessen obere Begrenzung durch die Schutzfläche 34 des Oberteils 31 ein, und dieser erreicht den Körper des Benutzers 1 erst nach Durchströmen des Bräunungsraums 32. So wird überraschenderweise nicht nur das störende Luft-Strömungsgeräusch (beispielsweise an den Ohren des Benutzers) vermieden, sondern auch eine als sanft und angenehm empfundene Kühlung bewirkt, die vom Benutzer nicht als Zug erfahren wird.

In einer weiter bevorzugten Ausführungsform der Erfindung sieht man die für zumindest bräunende UV-Strahlung durchlässige Schutzfläche 34 des Oberteils 31 zur Bildung des in Längsrichtung des Bräunungsgeräts 10 im wesentlichen tunnelförmigen Bräunungsraums 32 aus Sicht des Benutzers in konkav gewölbter Form vor. Die konkav gewölbte Schutzfläche ermöglicht in vorteilhafter Weise, daß der aus den Ausströmer-Düsen oder ―Kanälen 26 austretende Luftstrom entlang der konkaven Wölbung der Schutzfläche 34 strömen kann und dadurch gleichmäßig und gezielt wieder in den Bräunungsraum 32 zurückgeführt wird.

Erfindungsgemäß ist es weiter bevorzugt, für die für zumindest bräunende UV-Strahlung durchlässige Schutzfläche 34 des Oberteils 31 des Bräunungsgeräts 10 im Scheitel 36 der konkaven Wölbung in Längsrichtung des Oberteils 31 einen im Querschnitt keilförmigen Luft-Umlenker 38 vorzusehen. Der Luft-Umlenker 38 wird weiter bevorzugt aus einem Material hergestellt, das ― wie die Schutzfläche 34 für den Benutzer 1 ― für zumindest bräunende UV-Strahlung durchlässig ist, um zu verhindern, daß diese Einrichtung 38 einen Teil der von den Strahlern 35 emittierten bräunenden UV-Strahlung absorbiert oder abschirmt. Das Vorsehen eines Luft-Umlenkers 38 ist von besonderem Vorteil deswegen, weil sich die durch die Ausströmer-Düsen und/oder ―Kanäle 26 in den Bräunungsraum 32 eingespeiste Luft mit seiner Hilfe von der konkaven Wölbung der Schutzfläche 34 auf den Benutzer lenken läßt. In einer besonders bevorzugten Ausführungsform wird sogar die auf beiden Längsseiten 22a, 22b seitlich der Liegefläche durch die Ausströmer-Düsen und/oder -Kanäle 26 einströmende Luft mittels des Luft-Umlenkers 38 in der Mitte des Bräunungsraums 32 verwirbelt, was zu einem als besonders angenehm empfundenen temperierenden Luftstrom am Körper des Benutzers 1 führt.

In einer noch weiter bevorzugten, mit besonderem Vorteil verwirklichten Ausführungsform der Erfindung ist der Luft-Umlenker 38 Teil der für zumindest bräunende UV-Strahlung durchlässigen Schutzfläche 34. In diesem Fall wird natürlich der Luft-Umlenker 38 aus demselben Material wie die Schutzfläche 34 hergestellt und ist damit auch hinsichtlich seiner Durchlässigkeit für zumindest bräunende UV-Strahlung mit der Durchlässigkeit der Schutzfläche 34 identisch. Diese Ausführungsform ist auch aus Herstellungs-Sicht besonders vorteilhaft, da die Schutzfläche 34 zusammen mit dem Luft-Umlenker 38 integral gestaltet ist und entsprechend in einem Schritt hergestellt werden kann.

In einer dazu alternativen Ausführungsform kann der Luft-Umlenker 38 auf die für zumindest bräunende UV-Strahlung durchlässige Schutzfläche 34 aufgebracht werden, was natürlich auch nachträglich geschehen kann. Damit wird ein Nachrüsten der Schutzfläche 34 des Oberteils 32 des Bräunungsgeräts 10 mit dem Luft-Umlenker 38 möglich, so daß auch bereits laufenden Geräte die Vorteile der Lenkung des Luftstroms an der Schutzfläche 34 verliehen werden können. Die Aufbringung des Luft-Umlenkers 38 auf die Schutzfläche 34 kann auf jede beliebige, dem Fachmann bekannte Art und Weise erfolgen. Ganz besonders bevorzugt ist jedoch ein Aufkleben des Luft-Umlenkers 38 auf die Schutzfläche 34 des Oberteils 31 des Bräunungsgeräts 10.

Den Luft-Umlenker 38 auf der (oder integriert in die) für zumindest bräunende UV-Strahlung durchlässigen Schutzfläche 34 kann man mit einer völlig glatten Oberfläche vorsehen. Erfindungsgemäß ist es jedoch noch weiter bevorzugt, den Luft-Umlenker 38 mit einer der Verteilung und/oder Verwirbelung der strömenden Luft dienenden Oberflächenstruktur 39 zu versehen. Eine solche Oberflächenstruktur 39 kann zu einer noch besseren Verteilung des Luftstroms oder gar zu dessen Verwirbelung im Bräunungsraum 32 des Oberteils 31 des Bräunungsgeräts 10 führen, und die Temperierung des Bräunungsraums und des Körpers des Benutzers 1 erfolgen auf diesem Weg noch nachhaltiger und angenehmer. Die Oberflächenstruktur kann von beliebiger Art sein, wie sie der Fachmann für die angegebenen Zwecke kennt, ohne daß die Erfindung diesbezüglich irgendwelchen Beschränkungen unterliegt. In einer mit besonderem Vorteil verwirklichten Ausführungsform weist der Luft-Umlenker 38 Vertiefungen, Spiralen und/oder Wellen auf, die der Verteilung und/oder Verwirbelung der strömenden Luft dienen.

In einer alternativen oder auch zusammen mit der vorstehend beschriebenen Ausführungsform gemeinsam verwirklichten vorteilhaften Ausführungsform der Erfindung sieht man an dem Luft-Umlenker 38 Einrichtungen 40 vor, mit deren Hilfe die strömende Luft separat auf den Körper oder separat auf das Gesicht des Benutzers 1 geleitet werden kann. Solche Einrichtungen 40 sind dem Fachmann ebenfalls bekannt und können entweder statisch oder individuell einstellbar sein, damit in vorteilhafter Weise der Luftstrom auf die Stellen des Körpers oder des Gesichts gelenkt werden kann, die dem Benutzer 1 am angenehmsten sind.

In einer weiteren vorteilhaften Ausführungsform der Erfindung, die zusammen mit den vorstehenden Merkmalen des Bräunungsgeräts verwirklicht werden kann, sieht man an dem Unterteil 21 des Bräunungsgeräts 10 zwischen Liegefläche 24 und dem mindestens einen Satz Strahler 25 zur Abgabe von Strahlung mit zumindest einem Anteil an bräunender UV-Strahlung eine für zumindest bräunende UV-Strahlung durchlässige Zwischenfläche 28 vor, die im wesentlichen in einer Ebene parallel zur Ebene der Liegefläche 24 verläuft und die Führung von Luft im Raum zwischen der Zwischenfläche 28 und der Liegefläche 24 zur Temperierung der Liegefläche 24 erlaubt. Diese Ausführungsform der Erfindung trägt dem Erfordernis Rechnung, daß eine Temperierung der Liegefläche sowohl im Sinne einer Erwärmung bei kalter Liegefläche 24 als auch im Sinne einer Kühlung bei sich bei Betrieb der Strahler 25 erwärmender Liegefläche 24 erwünscht sein kann. Eine derartige Temperierung der Liegefläche 24 kann mittels Luft bewirkt werden, die eine für die Temperierung erforderliche Temperatur aufweist und die man durch den Raum zwischen der Zwischenfläche 28 und der Liegefläche 24 leitet.

Die Zwischenfläche 28 kann in dieser Ausführungsform der Erfindung aus demselben Material hergestellt werden wie die Liegefläche 24. Aufgrund der wesentlich geringeren mechanischen Anforderungen, verglichen mit der Liegefläche 24 ― die Zwischenfläche 28 wird gewichtsmäßig so gut wie gar nicht belastet ― kann jedoch die Zwischenfläche aus einem viel weniger starken Material oder aus einem völlig anderen Material hergestellt werden als die Liegefläche, vorausgesetzt, sie ist für zumindest bräunende UV-Strahlung hinreichend durchlässig. Eine 100 %ige Durchlässigkeit ist nicht erforderlich, eine möglichst hohe Durchlässigkeit für den bräunenden Anteil der UV-Strahlung ist jedoch erwünscht und angestrebt.

Der genaue Verlauf der Zwischenfläche 28, verglichen mit der Liegefläche 24, ist nicht von entscheidender Bedeutung, doch ist es bevorzugt, die Zwischenfläche 28 so anzuordnen, daß sie in einer Ebene im wesentlichen parallel zur Ebene der Liegefläche verläuft. Dies ist deswegen vorteilhaft, weil sich dadurch ein Raum zwischen der Zwischenfläche 28 und der Liegefläche 24 ergibt, der annährend gleiche Dicke hat, keine Luftstaus beim Durchströmen der temperierten Luft ergibt und damit eine angenehme und gezielte Temperierung der Liegefläche 24 ermöglicht.

Die Temperierung der Liegefläche mittels der durch den Raum zwischen Liegefläche 24 und Zwischenfläche 28 strömender Luft kann während des Betriebs des Bräunungsgeräts 10 erfolgen, wobei es in diesem Fall häufig um eine Kühlung der Liegefläche 24 gegen die von den Strahlern 25 emittierte Wärme geht, oder kann vor und/oder nach dem Betrieb des Bräunungsgeräts 10 erfolgen, wobei je nach Wunsch entweder eine Erwärmung oder eine Kühlung oder auch eines nach dem anderen (z. B. erst Erwärmung, dann Kühlung) erfolgen kann. So kann in besonders vorteilhafter Weise eine jeder Situation angepaßte und auf den individuellen Wunsch des Benutzers abgestellte Temperierung des Bräunungsgeräts 10, insbesondere der Liegefläche 24, gegebenenfalls zusammen mit einer Temperierung des Bräunungsraums erfolgen.

Die Temperierung der zum Temperieren verwendeten Luft kann auf jeden denkbare und dem Fachmann an sich bekannte Weise geschehen. Insbesondere können für die Temperierung technische Mittel verwendet werden, wie sie im Stand der Technik beschrieben sind, beispielsweise in der Druckschrift DE-A 100 53 900.9. In besonders bevorzugten Ausführungsformen des erfindungsgemäßen Verfahrens wird jedoch mittels eines separaten (vorzugsweise im Unterteil 21 des Bräunungsgeräts 10 untergebrachten) Lüfters die Luft in den Raum zwischen der Zwischenfläche 28 und der Liegefläche 24 eingespeist und die Liegefläche 24 mittels der durchströmenden (auf die gewünschte Temperatur gebrachten, also temperierten) Luft temperiert. Mit besonderem Vorteil sieht man dazu in dem erfindungsgemäßen Bräunungsgerät 10 den separate Lüfter mit einer (ebenfalls vorzugsweise im Unterteil 21 des Bräunungsgerät 10 untergebrachten) Kühl- oder Wärme-Einrichtung bzw. einem Klimagerät 30 verbunden vor. Vom Lüfter angeströmte Luft wird so in dem Klimagerät 30 temperiert und dann in den Raum zwischen der Zwischenfläche 28 und der Liegefläche 24 zur Temperierung der letzteren eingespeist. Dabei kann das Klimagerät 30 zum Erwärmen oder Kühlen der Luft Gebrauch von allen denkbaren Wärmequellen (z. B. den Strahlern 25) oder Wärmeverbrauchern (zur Erzeugung von "Kälte") machen, die im Stand der Technik für Erwärmungs- oder Kühlungszwecke, insbesondere bei Bräunungsgeräten, beschrieben sind.

Alternativ dazu oder in Ergänzung zu der vorher beschriebenen Ausführungsform entspricht es einer weiteren vorteilhaften Ausführungsform der Erfindung, wenn man die die Liegefläche 24 temperierende Luft über eine Passage 20 vom Bräunungsraum 32 in den Raum zwischen der Zwischenfläche 28 und der Liegefläche 24 führt. Damit wird die (beispielsweise kühlend) temperierende Luft des Bräunungsraums 32 anschließend auch für die Temperierung (z. B. Kühlung) der Liegefläche 24 genutzt, indem man sie aus dem Bräunungsraum 32 abzieht und über die Passage 20 unter der Liegefläche 24 hindurch strömen läßt. In einer besonders bevorzugten Ausführungsform geschieht dies in der Weise, daß die temperierende Luft vom Bräunungsraum 32 über eine kopfseitig und/oder fußseitig der Liegefläche 24 angeordnete Passage 20 in den Raum zwischen der Zwischenfläche 28 und der Liegefläche 24 geführt wird. Damit wird im wesentlichen eine vergleichbare Temperierung des Bräunungsraums 32 und der Liegefläche 24 erreicht, was für den Benutzer besonders angenehm ist.

In einer weiter bevorzugten Ausführungsform der erfindungsgemäßen Verfahren wird als Temperierung der (gegebenenfalls im Kreislauf geführte) Luftstrom gegenüber der das Bräunungsgerät 10 umgebenden Luft erwärmt. Noch mehr bevorzugt ist es, wenn der (gegebenenfalls im Kreislauf geführte) Luftstrom gegenüber der das Bräunungsgerät umgebenden Luft gekühlt wird.

Von großem praktischem Vorteil sind solche Verfahren gemäß der Erfindung, in denen man die Temperierung regelt, noch weiter bevorzugt in denen man die Temperierung über eine (an sich bekannte und noch mehr bevorzugt im Unterteil 21 des Bräunungsgerät 10 untergebrachte) Wärm- und/oder Kühleinrichtung 27a und/oder eine Einrichtung 27b zum Zudosieren von Umgebungsluft zum umlaufenden Luftstrom regelt. So werden kurze, keine Wärme- oder "Kälte"-Verluste bedingende Kreisläufe geschaffen, die mit einfachen, zum Teil mechanischen Mitteln wie Klappen zu regeln sind, die gegebenenfalls mit einer Temperatursteuerung verbunden werden können.

Mit den vorbeschriebenen einzelnen Merkmalen oder einer Kombination beliebiger Merkmale der Erfindung, insbesondere den bevorzugten Merkmalen der Erfindung, wird eine für den Benutzer 1 angenehme Temperierung des Bräunungsraums 32, des Körpers des Benutzers 1 und gegebenenfalls auch der Liegefläche 24 erreicht. Die hierfür erforderlichen Einrichtungen befinden sich im wesentlichen im Unterteil 21 des Bräunungsgeräts 10 und belasten dadurch das Oberteil 31 kaum. Weiter wird eine zugfreie Temperierung des Körpers des Benutzers 1 im Bräunungsraum 32 ermöglicht, wodurch das Bräunungserlebnis intensiviert und auch ein Wahrnehmen akustischer Signale (z. B. Musik) nicht durch Luft-Strömungsgeräusche gestört wird.

## Patentansprüche

1. Bräunungsgerät (10), insbesondere zur Ganzkörper-Bräunung eines Benutzers (1), umfassend
- ein standfestes Unterteil (21), enthaltend elektrische Aggregate, Schalt-Einrichtungen und Verbindungs-Einrichtungen; und
- ein entweder an einer der Längsseiten (22) des Unterteils (21) über Scharniere oder Gelenke (23) angebrachtes, über die Scharniere oder Gelenke (23) von dem Unterteil (21) weg und auf das Unterteil (21) hin zur Bildung eines im wesentlichen tunnelförmigen Bräunungsraumes (32) klappbares oder schwenkbares oder an einer separaten Aufhängung (33) von dem Unterteil (21) weg hebbares und auf das Unterteil (21) hin zur Bildung eines im wesentlichen tunnelförmigen Bräunungsraumes (32) senkbares Oberteil (31); wobei
- das Unterteil (21) eine für zumindest bräunende UV-Strahlung durchlässige Liegefläche (24) für den Benutzer (1) und unterhalb der Liegefläche (24) mindestens einen Satz in Längsrichtung des Bräunungsgeräts (10) angeordneter Strahler (25) zur Abgabe von Strahlung mit zumindest einem Anteil an bräunender UV-Strahlung in Richtung auf das Oberteil (31) und/oder zumindest einen Teil des Körpers des Benutzers (1) gerichtet mit den elektrischen Aggregaten, Schalt-Einrichtungen und/oder Verbindungs-Einrichtungen direkt oder indirekt verbunden aufweist; und
- das Oberteil (31) eine für zumindest bräunende UV-Strahlung durchlässige Schutzfläche (34) für den Benutzer (1) und oberhalb der Schutzfläche (34) mindestens einen Satz in Längsrichtung des Bräunungsgeräts (10) angeordneter Strahler (35) zur Abgabe von Strahlung mit zumindest einem Anteil an bräunender UV-Strahlung in Richtung auf das Unterteil (21) und/oder zumindest einen Teil des Körpers des Benutzers (1) gerichtet aufweist; und wobei
- das Unterteil (21) entlang zumindest eines Teils der Längsseiten (22a, 22b) der Liegefläche (24) Ausströmer-Düsen (26) oder Ausströmer-Kanäle (26) aufweist, die im wesentlichen in Richtung auf die Schutzfläche (34) des Oberteils (31) gerichtet sind und in den Bräunungsraum (32) einen Luftstrom einspeisen, der den Körper des Benutzers (1) nach Durchströmen des Bräunungsraums (32) temperiert.

2. Bräunungsgerät (10) nach Anspruch 1, wobei die für zumindest bräunende UV-Strahlung durchlässige Schutzfläche (34) des Oberteils (31) für den Benutzer (1) zur Bildung des in Längsrichtung des Bräunungsgeräts (10) im wesentlichen tunnelförmigen Bräunungsraums (32) aus Sicht des Benutzers (1) konkav gewölbt ist.

3. Bräunungsgerät (10) nach Anspruch 1 oder Anspruch 2, wobei die für zumindest bräunende UV-Strahlung durchlässige Schutzfläche (34) des Oberteils (31) für den Benutzer (1) im Scheitel (36) der konkaven Wölbung in Längsrichtung des Oberteils (31) einen im Querschnitt keilförmigen Luft-Umlenker (38) aufweist, vorzugsweise einen im Querschnitt keilförmigen, für UV-Strahlung durchlässigen Luft-Umlenker (38) aufweist.

4. Bräunungsgerät (10) nach einem der Ansprüche 1 bis 3, wobei der Luft-Umlenker (38) Teil der für zumindest bräunende UV-Strahlung durchlässigen Schutzfläche (34) ist.

5. Bräunungsgerät (10) nach einem der Ansprüche 1 bis 3, wobei der Luft-Umlenker (38) auf die für zumindest bräunende UV-Strahlung durchlässige Schutzfläche (34) aufgebracht ist, vorzugsweise auf die für zumindest bräunende UV-Strahlung durchlässige Schutzfläche (34) aufgeklebt ist.

6. Bräunungsgerät (10) nach einem der Ansprüche 1 bis 5, wobei der Luft-Umlenker (38) eine der Verteilung und/oder Verwirbelung der strömenden Luft dienende Oberflächenstruktur (39) aufweist, vorzugsweise worin der Luft-Umlenker (38) Vertiefungen, Spiralen oder Wellen zur Verteilung und/oder Verwirbelung der strömenden Luft aufweist.

7. Bräunungsgerät (10) nach einem der Ansprüche 1 bis 6, wobei der Luft-Umlenker (38) Einrichtungen (40) zum Leiten der strömenden Luft separat auf den Körper oder separat auf das Gesicht des Benutzers (1) aufweist.

8. Bräunungsgerät (10) nach einem der Ansprüche 1 bis 7, wobei das Unterteil (21) entlang beider Längsseiten (22a, 22b) der Liegefläche (24) Ausströmer-Düsen (26) oder Ausströmer-Kanäle (26) aufweist, vorzugsweise worin das Unterteil (21) über die gesamte Länge beider Längsseiten (22a, 22b) je einen Ausströmer-Kanal (26) aufweist.

9. Bräunungsgerät (10) nach einem der Ansprüche 1 bis 8, wobei das Unterteil (21) zwischen Liegefläche (24) und dem mindestens einen Satz Strahler (25) zur Abgabe von Strahlung mit zumindest einem Anteil an bräunender UV-Strahlung eine für zumindest bräunende UV-Strahlung durchlässige Zwischenfläche (28) aufweist, die im wesentlichen in einer Ebene parallel zur Ebene der Liegefläche (24) verläuft und die Führung von Luft im Raum zwischen der Zwischenfläche (28) und der Liegefläche (24) zur Temperierung der letzteren erlaubt.

10. Bräunungsgerät (10) nach Anspruch 9, wobei ein separater Lüfter im Unterteil (21) die Luft in den Raum zwischen der Zwischenfläche (28) und der Liegefläche (24) zur Temperierung der letzteren einspeist, vorzugsweise wobei ein separater Lüfter in Verbindung mit einem Klimagerät (30) oder einer Kühleinrichtung (27a) temperierte Luft in den Raum zwischen der Zwischenfläche (28) und der Liegefläche (24) zur Temperierung der letzteren einspeist.

11. Bräunungsgerät (10) nach Anspruch 9 oder Anspruch 10, wobei die temperierende Luft über eine Passage (20) vom Bräunungsraum (32) in den Raum zwischen der Zwischenfläche (28) und der Liegefläche (24) geführt ist, vorzugsweise worin die temperierende Luft vom Bräunungsraum (32) über eine kopfseitig oder fußseitig der Liegefläche (24) angeordnete Passage (20) in den Raum zwischen der Zwischenfläche (28) und der Liegefläche (24) geführt ist.

12. Bräunungsgerät (10), insbesondere zur Ganzkörper-Bräunung eines Benutzers (1), umfassend
- ein standfestes Unterteil (21), enthaltend elektrische Aggregate, Schalt-Einrichtungen und Verbindungs-Einrichtungen; und
- ein entweder an einer der Längsseiten (22) des Unterteils (21) über Scharniere oder Gelenke (23) angebrachtes, über die Scharniere oder Gelenke (23) von dem Unterteil (21) weg und auf das Unterteil (21) hin zur Bildung eines im wesentlichen tunnelförmigen Bräunungsraumes (32) klappbares oder schwenkbares oder an einer separaten Aufhängung (33) von dem Unterteil (21) weg hebbares und auf das Unterteil (21) hin zur Bildung eines im wesentlichen tunnelförmigen Bräunungsraumes (32) senkbares Oberteil (31); wobei
- das Unterteil (21) eine für zumindest bräunende UV-Strahlung durchlässige Liegefläche (24) für den Benutzer (1) und unterhalb der Liegefläche (24) mindestens einen Satz in Längsrichtung des Bräunungsgeräts (10) angeordneter Strahler (25) zur Abgabe von Strahlung mit zumindest einem Anteil an bräunender UV-Strahlung in Richtung auf das Oberteil (31) und/oder zumindest einen Teil des Körpers des Benutzers (1) gerichtet mit den elektrischen Aggregaten, Schalt-Einrichtungen und/oder Verbindungs-Einrichtungen direkt oder indirekt verbunden aufweist; und
- das Oberteil (31) eine für zumindest bräunende UV-Strahlung durchlässige Schutzfläche (34) für den Benutzer (1) und oberhalb der Schutzfläche (34) mindestens einen Satz in Längsrichtung des Bräunungsgeräts (10) angeordneter Strahler (35) zur Abgabe von Strahlung mit zumindest einem Anteil an bräunender UV-Strahlung in Richtung auf das Unterteil (21) und/oder zumindest einen Teil des Körpers des Benutzers (1) gerichtet aufweist; und wobei
- das Unterteil (21) zwischen Liegefläche (24) und dem mindestens einen Satz Strahler (25) zur Abgabe von Strahlung mit zumindest einem Anteil an bräunender UV-Strahlung eine für zumindest bräunende UV-Strahlung durchlässige Zwischenfläche (28) aufweist, die im wesentlichen in einer Ebene parallel zur Ebene der Liegefläche (24) verläuft und die Führung von Luft im Raum zwischen der Zwischenfläche (28) und der Liegefläche (24) zur Temperierung der letzteren erlaubt.

13. Bräunungsgerät (10) nach Anspruch 12, wobei das Unterteil (21) entlang zumindest eines Teils der Längsseiten (22a, 22b) der Liegefläche (24) Ausströmer-Düsen (26) oder Ausströmer-Kanäle (26) aufweist, die im wesentlichen in Richtung auf die Schutzfläche (34) des Oberteils (31) gerichtet sind und in den Bräunungsraum (32) einen Luftstrom einspeisen, der den Körper des Benutzers (1) nach Durchströmen des Bräunungsraums (32) temperiert.

14. Bräunungsgerät (10) nach Anspruch 12 oder Anspruch 13, wobei die für zumindest bräunende UV-Strahlung durchlässige Schutzfläche (34) des Oberteils (31) für den Benutzer (1) zur Bildung des in Längsrichtung des Bräunungsgeräts (10) im wesentlichen tunnelförmigen Bräunungsraums (32) aus Sicht des Benutzers (1) konkav gewölbt ist.

15. Bräunungsgerät (10) nach einem der Ansprüche 12 bis 14, wobei die für zumindest bräunende UV-Strahlung durchlässige Schutzfläche (34) des Oberteils (31) für den Benutzer (1) im Scheitel (36) der konkaven Wölbung in Längsrichtung des Oberteils (31) einen im Querschnitt keilförmigen Luft-Umlenker (38) aufweist, vorzugsweise einen im Querschnitt keilförmigen, für UV-Strahlung durchlässigen Luft-Umlenker (38) aufweist.

16. Bräunungsgerät (10) nach einem der Ansprüche 12 bis 15, wobei der Luft-Umlenker (38) Teil der zumindest für bräunende UV-Strahlung durchlässigen Schutzfläche (34) ist.

17. Bräunungsgerät (10) nach einem der Ansprüche 12 bis 15, wobei der Luft-Umlenker (38) auf die zumindest für bräunende UV-Strahlung durchlässige Schutzfläche (34) aufgebracht ist, vorzugsweise auf die zumindest für bräunende UV-Strahlung durchlässige Schutzfläche (34) aufgeklebt ist.

18. Bräunungsgerät (10) nach einem der Ansprüche 12 bis 17, wobei der Luft-Umlenker (38) eine der Verteilung und/oder Verwirbelung der strömenden Luft dienende Oberflächenstruktur (39) aufweist, vorzugsweise worin der Luft-Umlenker (38) Vertiefungen, Spiralen oder Wellen zur Verteilung und/oder Verwirbelung der strömenden Luft aufweist.

19. Bräunungsgerät (10) nach einem der Ansprüche 12 bis 18, wobei der Luft-Umlenker (38) Einrichtungen (40) zum Leiten der strömenden Luft separat auf den Körper oder separat auf das Gesicht des Benutzers (1) aufweist.

20. Bräunungsgerät (10) nach einem der Ansprüche 12 bis 19, wobei das Unterteil (21) entlang beider Längsseiten (22a, 22b) der Liegefläche (24) Ausströmer-Düsen (26) oder Ausströmer-Kanäle (26) aufweist, vorzugsweise worin das Unterteil (21) über die gesamte Länge beider Längsseiten (22a, 22b) je einen Ausströmer-Kanal (26) aufweist.

21. Bräunungsgerät (10) nach einem der Ansprüche 12 bis 20, wobei ein separater Lüfter (29) im Unterteil (21) die Luft in den Raum zwischen der Zwischenfläche (28) und der Liegefläche (24) zur Temperierung der letzteren einspeist, vorzugsweise worin ein separater Lüfter (29) in Verbindung mit einem Klimagerät (30) oder einer Kühleinrichtung (27a) temperierte Luft in den Raum zwischen der Zwischenfläche (28) und der Liegefläche (24) zur Temperierung der letzteren einspeist.

22. Bräunungsgerät (10) nach einem der Ansprüche 12 bis 21, wobei die temperierende Luft über eine Passage (20) vom Bräunungsraum (32) in den Raum zwischen der Zwischenfläche (28) und der Liegefläche (24) geführt ist, vorzugsweise worin die temperierende Luft vom Bräunungsraum (32) über eine kopfseitig oder fußseitig der Liegefläche (24) angeordnete Passage (20) in den Raum zwischen der Zwischenfläche (28) und der Liegefläche (24) geführt ist.

23. Bräunungsgerät (10) zur Ganzkörper-Bräunung eines Benutzers (1), umfassend
- ein standfestes Unterteil (21), enthaltend elektrische Aggregate, Schalt-Einrichtungen und Verbindungs-Einrichtungen; und
- ein entweder an einer der Längsseiten (22) des Unterteils (21) über Scharniere oder Gelenke (23) angebrachtes, über die Scharniere oder Gelenke (23) von dem Unterteil (21) weg und auf das Unterteil (21) hin zur Bildung eines im wesentlichen tunnelförmigen Bräunungsraumes (32) klappbares oder schwenkbares oder an einer separaten Aufhängung (33) von dem Unterteil (21) weg hebbares und auf das Unterteil (21) hin zur Bildung eines im wesentlichen tunnelförmigen Bräunungsraumes (32) senkbares Oberteil (31); wobei
- das Unterteil (21) eine für zumindest bräunende UV-Strahlung durchlässige Liegefläche (24) für den Benutzer (1) und unterhalb der Liegefläche (24) mindestens einen Satz in Längsrichtung des Bräunungsgeräts (10) angeordneter Strahler (25) zur Abgabe von Strahlung mit zumindest einem Anteil an bräunender UV-Strahlung in Richtung auf das Oberteil (31) und/oder zumindest einen Teil des Körpers des Benutzers (1) gerichtet mit den elektrischen Aggregaten, Schalt-Einrichtungen und/oder Verbindungs-Einrichtungen direkt oder indirekt verbunden aufweist; und
- das Oberteil (31) eine für zumindest bräunende UV-Strahlung durchlässige Schutzfläche (34) für den Benutzer (1) und oberhalb der Schutzfläche (34) mindestens einen Satz in Längsrichtung des Bräunungsgeräts (10) angeordneter Strahler (35) zur Abgabe von Strahlung mit zumindest einem Anteil an bräunender UV-Strahlung in Richtung auf das Unterteil (21) und/oder zumindest einen Teil des Körpers des Benutzers (1) gerichtet aufweist; und wobei
- das Unterteil (21) entlang zumindest eines Teils der Längsseiten (22a, 22b) der Liegefläche (24) Ausströmer-Düsen (26) oder Ausströmer-Kanäle (26) aufweist, die im wesentlichen in Richtung auf die Schutzfläche (34) des Oberteils (31) gerichtet sind und in den Bräunungsraum (32) einen Luftstrom einspeisen, der den Bräunungsraum (32) auch bei nicht arbeitenden Strahlern (25, 35) temperiert.

24. Bräunungsgerät (10) nach Anspruch 23, wobei der eingespeiste Luftstrom nach Durchströmen des Bräunungsraums (32) kopfseitig oder fußseitig abgesaugt und einem temperierenden Klimagerät (30) zugeführt wird, das den temperierten Luftstrom wieder in den Bräunungsraum (32) einspeist.

25. Bräunungsgerät (10) nach einem der Ansprüche 23 oder 24, wobei die Kopfund/oder die Fußseite des Bräunungsraums (32) des Bräunungsgeräts (10) jeweils eine entfernbare Wand (29a, 29b) zum Verschließen des Bräunungsraums (32) aufweisen.

26. Bräunungsgerät (10) nach einem der Ansprüche 23 bis 25, wobei die für zumindest bräunende UV-Strahlung durchlässige Schutzfläche (34) des Oberteils (31) für den Benutzer (1) zur Bildung des in Längsrichtung des Bräunungsgeräts (10) im wesentlichen tunnelförmigen Bräunungsraums (32) aus Sicht des Benutzers (1) konkav gewölbt ist.

27. Bräunungsgerät (10) nach einem der Ansprüche 23 bis 26, wobei die für zumindest bräunende UV-Strahlung durchlässige Schutzfläche (34) des Oberteils (31) für den Benutzer (1) im Scheitel (36) der konkaven Wölbung in Längsrichtung des Oberteils (31) einen im Querschnitt keilförmigen Luft-Umlenker (38) aufweist, vorzugsweise einen im Querschnitt keilförmigen, für UV-Strahlung durchlässigen Luft-Umlenker (38) aufweist.

28. Bräunungsgerät (10) nach einem der Ansprüche 23 bis 27, wobei der Luft-Umlenker (38) Teil der zumindest für bräunende UV-Strahlung durchlässigen Schutzfläche (34) ist.

29. Bräunungsgerät (10) nach einem der Ansprüche 23 bis 27, wobei der Luft-Umlenker (38) auf die zumindest für bräunende UV-Strahlung durchlässige Schutzfläche (34) aufgebracht ist, vorzugsweise auf die zumindest für bräunende UV-Strahlung durchlässige Schutzfläche (34) aufgeklebt ist.

30. Bräunungsgerät (10) nach einem der Ansprüche 23 bis 29, wobei der Luft-Umlenker (38) eine der Verteilung und/oder Verwirbelung der strömenden Luft dienende Oberflächenstruktur (39) aufweist, vorzugsweise worin der Luft-Umlenker (38) Vertiefungen, Spiralen oder Wellen zur Verteilung und/oder Verwirbelung der strömenden Luft aufweist.

31. Bräunungsgerät (10) nach einem der Ansprüche 23 bis 30, wobei der Luft-Umlenker (38) Einrichtungen (40) zum Leiten der strömenden Luft separat auf den Körper oder separat auf das Gesicht des Benutzers (1) aufweist.

32. Bräunungsgerät (10) nach einem der Ansprüche 23 bis 31, wobei das Unterteil (21) entlang beider Längsseiten (22a, 22b) der Liegefläche (24) Ausströmer-Düsen (26) oder Ausströmer-Kanäle (26) aufweist, vorzugsweise worin das Unterteil (21) über die gesamte Länge beider Längsseiten (22a, 22b) je einen Ausströmer-Kanal (26) aufweist.

33. Bräunungsgerät (10) nach einem der Ansprüche 23 bis 32, wobei das Unterteil (21) zwischen Liegefläche (24) und dem mindestens einen Satz Strahler (25) zur Abgabe von Strahlung mit zumindest einem Anteil an bräunender UV-Strahlung eine für zumindest bräunende UV-Strahlung durchlässige Zwischenfläche (28) aufweist, die im wesentlichen in einer Ebene parallel zur Ebene der Liegefläche (24) verläuft und die Führung von Kühl-Luft im Raum zwischen der Zwischenfläche (28) und der Liegefläche (24) zur Kühlung der letzteren erlaubt.

34. Bräunungsgerät (10) nach einem der Ansprüche 23 bis 33, wobei ein separater Lüfter (29) im Unterteil (21) die Luft in den Raum zwischen der Zwischenfläche (28) und der Liegefläche (24) zur Kühlung der letzteren einspeist, vorzugsweise worin ein separater Lüfter (29) in Verbindung mit einem Klimagerät (30) oder einer Kühleinrichtung (27a) gekühlte Luft in den Raum zwischen der Zwischenfläche (28) und der Liegefläche (24) zur Kühlung der letzteren einspeist.

35. Bräunungsgerät (10) nach einem der Ansprüche 23 bis 34, wobei die Kühl-Luft über eine Passage (20) vom Bräunungsraum (32) in den Raum zwischen der Zwischenfläche (28) und der Liegefläche (24) geführt ist, vorzugsweise worin die Kühl-Luft vom Bräunungsraum (32) über eine kopfseitig oder fußseitig der Liegefläche (24) angeordnete Passage (20) in den Raum zwischen der Zwischenfläche (28) und der Liegefläche (24) geführt ist.

36. Bräunungsgerät (10) nach einem der Ansprüche 1 bis 35, wobei die Temperierung in einer Erwärmung oder vorzugsweise einer Kühlung des Luftstroms gegenüber der das Bräunungsgerät (10) umgebenden Luft besteht.

37. Bräunungsgerät (10) nach einem der Ansprüche 1 bis 36, wobei die Temperierung regelbar ist, vorzugsweise wobei die Temperierung über eine Wärmund/oder Kühl-Einrichtung (27a) und eine Einrichtung (27b) zum Zudosieren von Umgebungsluft zum umlaufenden Luftstrom regelbar ist.

38. Verfahren zum Temperieren eines Bräunungsraums (32) eines Bräunungsgeräts (10) mit
- einem standfesten Unterteil (21), enthaltend elektrische Aggregate, Schalt-Einrichtungen und Verbindungs-Einrichtungen; und
- einem entweder an einer der Längsseiten (22) des Unterteils (21) über Scharniere oder Gelenke (23) angebrachten, über die Scharniere oder Gelenke (23) von dem Unterteil (21) weg und auf das Unterteil (21) hin zur Bildung eines im wesentlichen tunnelförmigen Bräunungsraumes (32) klappbaren oder schwenkbaren oder an einer separaten Aufhängung (33) von dem Unterteil (21) weg hebbaren und auf das Unterteil (21) hin zur Bildung eines im wesentlichen tunnelförmigen Bräunungsraumes (32) senkbaren Oberteil (31); wobei
- das Unterteil (21) eine für zumindest bräunende UV-Strahlung durchlässige Liegefläche (24) für den Benutzer (1) und unterhalb der Liegefläche (24) mindestens einen Satz in Längsrichtung des Bräunungsgeräts (10) angeordneter Strahler (25) zur Abgabe von Strahlung mit zumindest einem Anteil an bräunender UV-Strahlung in Richtung auf das Oberteil (31) und/oder zumindest einen Teil des Körpers des Benutzers (1) gerichtet mit den elektrischen Aggregaten, Schalt-Einrichtungen und/oder Verbindungs-Einrichtungen direkt oder indirekt verbunden aufweist; und
- das Oberteil (31) eine für zumindest bräunende UV-Strahlung durchlässige Schutzfläche (34) für den Benutzer (1) und oberhalb der Schutzfläche (34) mindestens einen Satz in Längsrichtung des Bräunungsgeräts (10) angeordneter Strahler (35) zur Abgabe von Strahlung mit zumindest einem Anteil an bräunender UV-Strahlung in Richtung auf das Unterteil (21) und/oder zumindest einen Teil des Körpers des Benutzers (1) gerichtet aufweist;
- bei dem man mittels entlang zumindest der Längsseiten (22a, 22b) der Liegefläche (24) des Unterteils (21) des Bräunungsgeräts (10) angeordneter Ausströmer-Düsen (26) oder Ausströmer-Kanäle (26) im wesentlichen in Richtung auf die Schutzfläche (34) des Oberteils (31) des Bräunungsgeräts (10) in den Bräunungsraum (32) einen Luftstrom einspeist, der den Bräunungsraum (32) bei Durchströmen temperiert.

39. Verfahren zum Temperieren des Körpers eines Benutzers (1) in einem Bräunungsgerät (10) mit
- einem standfesten Unterteil (21), enthaltend elektrische Aggregate, Schalt-Einrichtungen und Verbindungs-Einrichtungen; und
- einem entweder an einer der Längsseiten (22) des Unterteils (21) über Scharniere oder Gelenke (23) angebrachten, über die Scharniere oder Gelenke (23) von dem Unterteil (21) weg und auf das Unterteil (21) hin zur Bildung eines im wesentlichen tunnelförmigen Bräunungsraumes (32) klappbaren oder schwenkbaren oder an einer separaten Aufhängung (33) von dem Unterteil (21) weg hebbaren und auf das Unterteil (21) hin zur Bildung eines im wesentlichen tunnelförmigen Bräunungsraumes (32) senkbaren Oberteil (31); wobei
- das Unterteil (21) eine für zumindest bräunende UV-Strahlung durchlässige Liegefläche (24) für den Benutzer (1) und unterhalb der Liegefläche (24) mindestens einen Satz in Längsrichtung des Bräunungsgeräts (10) angeordneter Strahler (25) zur Abgabe von Strahlung mit zumindest einem Anteil an bräunender UV-Strahlung in Richtung auf das Oberteil (31) und/oder zumindest einen Teil des Körpers des Benutzers (1) gerichtet mit den elektrischen Aggregaten, Schalt-Einrichtungen und/oder Verbindungs-Einrichtungen direkt oder indirekt verbunden aufweist; und
- das Oberteil (31) eine für zumindest bräunende UV-Strahlung durchlässige Schutzfläche (34) für den Benutzer (1) und oberhalb der Schutzfläche (34) mindestens einen Satz in Längsrichtung des Bräunungsgeräts (10) angeordneter Strahler (35) zur Abgabe von Strahlung mit zumindest einem Anteil an bräunender UV-Strahlung in Richtung auf das Unterteil (21) und/oder zumindest einen Teil des Körpers des Benutzers (1) gerichtet aufweist;
- bei dem man mittels entlang zumindest der Längsseiten (22a, 22b) der Liegefläche (24) des Unterteils (21) des Bräunungsgeräts (10) angeordneter Ausströmer-Düsen (26) oder Ausströmer-Kanäle (26) im wesentlichen in Richtung auf die Schutzfläche (34) des Oberteils (31) des Bräunungsgeräts (10) in den Bräunungsraum (32) einen Luftstrom einspeist, der den Körper des Benutzers (1) nach Durchströmen des Bräunungsraums (32) temperiert.

40. Verfahren nach den Ansprüchen 38 oder 39, wobei als Temperierung der Luftstrom gegenüber der das Bräunungsgerät (10) umgebenden Luft erwärmt oder vorzugsweise gekühlt wird.

41. Verfahren nach einem der Ansprüche 38 bis 40, wobei man die Temperierung regelt, vorzugsweise wobei man die Temperierung über eine Wärm- und/oder Kühl-Einrichtung (27a) und eine Einrichtung (27b) zum Zudosieren von Umgebungsluft zum umlaufenden Luftstrom regelt.
